(19) Europäisches Patentamt
European Patent Office
Office européen des brevets

(11) EP 0 950 657 B1

(12) **EUROPEAN PATENT SPECIFICATION**

(45) Date of publication and mention of the grant of the patent:
**14.07.2004 Bulletin 2004/29**

(51) Int Cl.[7]: **C07D 209/88**, C07D 209/86, A61K 31/40

(21) Application number: **99302967.7**

(22) Date of filing: **16.04.1999**

(54) **Substituted carbazoles, process for their preparation and their use as sPLA2 inhibitors**

Substituierte Karbazole, Verfahren zu ihrer Herstellung und ihre Verwendung als sPLA2-Inhibitoren

Carbazoles substitués, procédé pour leur préparation et leur utilisation comme inhibiteurs de l' enzyme sPLA2

(84) Designated Contracting States:
**AT BE CH CY DE DK ES FI FR GB GR IE IT LI LU NL PT SE**
Designated Extension States:
**AL LT LV RO SI**

(30) Priority: **17.04.1998 US 62328**

(43) Date of publication of application:
**20.10.1999 Bulletin 1999/42**

(73) Proprietor: **ELI LILLY AND COMPANY**
**Indianapolis, Indiana 46285 (US)**

(72) Inventors:
- **Bach, Nicholas James**
**Indianapolis, Indiana 46217 (US)**
- **Bastian, Jolie Anne**
**Beech Grove, Indiana 46107 (US)**
- **Hite, Gary Alan**
**Indianapolis, Indiana 46227 (US)**
- **Kinnick, Michael Dean**
**South Indianapolis, Indiana 46217 (US)**
- **Mihelich, Edward David**
**Carmel, Indiana 46032 (US)**
- **Morin, John Michael Jr.**
**Brownsburg, Indiana 46112 (US)**
- **Sall, Daniel Jon**
**Greenwood, Indiana 46142 (US)**
- **Vasileff, Robert Theodore**
**Indianapolis, Indiana 46228 (US)**

(74) Representative: **Vaughan, Jennifer Ann et al**
**Lilly Research Centre,**
**Erl Wood Manor**
**Windlesham, Surrey GU20 6PH (GB)**

(56) References cited:
**EP-A- 0 620 214**
**EP-A- 0 620 215**
**EP-A- 0 749 962**
**EP-A- 0 779 271**
**EP-A- 0 839 806**
**US-A- 3 979 391**
**US-A- 5 420 289**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 950 657 B1

Printed by Jouve, 75001 PARIS (FR)

## Description

This application claims the benefit of U. S. Serial No. 09/062,328 filed April 17, 1998.

This invention relates to novel substituted tricyclic organic compounds useful for inhibiting $sPLA_2$ mediated release of fatty acids for conditions such as septic shock.

The structure and physical properties of human non-pancreatic secretory phospholipase $A_2$ (hereinafter called, "$sPLA_2$") has been thoroughly described in two articles, namely, "Cloning and Recombinant Expression of Phospholipase $A_2$ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase $A_2$" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that $sPLA_2$ is a rate limiting enzyme in the arachidonic acid cascade which hydrolyzes membrane phospholipids. Thus, it is important to develop compounds which inhibit $sPLA_2$ mediated release of fatty acids (e.g., arachidonic acid). Such compounds would be of value in general treatment of conditions induced and/or maintained by overproduction of $sPLA_2$ such as septic shock, adult respiratory distress syndrome, pancreatitis, trauma-induced shock, bronchial asthma, allergic rhinitis, rheumatoid arthritis, etc.

It is desirable to develop new compounds and treatments for $sPLA_2$ induced diseases.

The compounds contemplated by this invention are selected from the group consisting of [9-benzyl-5-carbamoyl-1-fluorocarbazol-4-yl]oxyacetic acid, {9-[(phenyl)methyl]-5-carbamoylcarbazol-4-yl)oxyacetic acid, {9-[(3-fluorophenyl)methyl]-5-carbamoylcarbazol-4-yl]oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl}oxyacetic acid, sodium salt, {9-[(2-methylphenyl)methyl]-5-carbamaylcarbazol-4-yl}oxyacetic acid, sodium salt, {9-[(3-methylphenyl)methyl]-5-carbamoylcarbazol-4-yl}oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl]oxyacetic acid, [9-[(cyclopentyl)methyl]-5-carbamoylcarbazol-4-yl]oxyacetic acid or a pharmaceutically acceptable racemate, solvate, tautomer, optical isomer, prodrug derivative or salt thereof.

This invention is also a pharmaceutical formulation comprising a compound selected from the group consisting of [9-benzyl-5-carbamoyl-1-fluorocarbazol-4-yl]oxyacetic acid, {9-[(phenyl)methyl]-5-carbamoylcarbazol-4-yl}oxyacetic acid, {9-[(3-fluorophenyl)methyl]-5-carbamoylcarbazol-4-yl}oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl}oxyacetic acid, sodium salt, {9-[(2-methylphenyl)methyl]-5-carbamoylcarbazol-4-yl}oxyacetic acid, sodium salt, {9-[(3-methylphenyl)methyl]-5-carbamoylcarbazol-4-yl}oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl]oxyacetic acid, and [9-[(cyclopentyl)methyl]-5-carbamoylcarbazol-4-yl]oxyacetic acid in association with one or more pharmaceutically acceptable diluents, carriers and excipients.

This invention is also the use of a compound selected from the group consisting of [9-benzyl-5-carbamoyl-1-fluorocarbazol-4-yl]oxyacetic acid, {9-[(phenyl)methyl]-5-carbamoylcarbazol-4-yl}oxyacetic acid, {9-[(3-fluorophenyl)methyl]-5-carbamoylcarbazol-4-yl}oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl}oxyacetic acid, sodium salt, {9-[(2-methylphenyl) methyl]-5-carbamoylcarbazol-4-yl}oxyacetic acid, sodium salt, {9-[(3-methylphenyl)methyl]-5-carbamoylcarbazol-4-yl} oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl] oxyacetic acid, and [9-[(cyclopentyl)methyl]-5-carbamoylcarbazol-4-yl]oxyacetic acid for the manufacture of a medicament for inhibiting $sPLA_2$ in a mammal.

According to a further aspect of the present invention, there is provided the use of a compound selected from the group consisting of [9-benzyl-5-carbamoyl-1-fluorocarbazol-4-yl]oxyacetic acid, {9-[(phenyl)methyl]-5-carbamoylcarbazol-4-yl}oxyacetic acid, {9-[(3-fluorophenyl)methyl]-5-carbamoylcarbazol-4-yl}oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl} oxyacetic acid, sodium salt, {9-[(2-methylphenyl)methyl]-5-carbamoylcarbazol-4-yl}oxyacetic acid, sodium salt, {9-[(3-methylphenyl)methyl]-5-carbamoylcarbazol-4-yl}oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl]oxyacetic acid, and [9-[(cyclopentyl)methyl]-5-carbamoylcarbazol-4-yl]oxyacetic acid for the manufacture of a medicament for selectively inhibiting $sPLA_2$ in a mammal.

This invention, further provides a compound selected from the group consisting of [9-benzyl-5-carbamoyl-1-fluorocarbazol-4-yl]oxyacetic acid, {9-[(phenyl)methyl]-5-carbamoylcarbazol-4-yl}oxyacetic acid I, {9-[(3-fluorophenyl)methyl]-5-carbamoylcarbazol-4-yl}oxyacetic acid, {9-[(3-chlorophenyl)methyl]-5-carbamoylcarbazol-4-yl}oxyacetic

acid, {9-[(3-trifluoromethylphenyl)methyl]-5-carbamoylcarbazol-4-yl}oxyacetic acid, sodium salt, {9-[(2-methylphenyl) methyl]-5-carbamoylcarbazol-4-yl}oxyacetic acid, sodium salt, {9-[(3-methylphenyl)methyl]-5-carbamoylcarbazol-4-yl} oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl] oxyacetic acid, and [9-[(cyclopentyl)methyl]-5-carbamoylcarbazol-4-yl]oxyacetic acid for use as a medicament in the treatment of inflammatory diseases such as, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases which comprises administering to a mammal in need of such treatment a therapeutically effective amount of the compound of formula I in an amount sufficient to inhibit $sPLA_2$ mediated release of fatty acid and to thereby inhibit or prevent the arachidonic acid cascade and its deleterious products.

Other objects, features and advantages of the present invention will become apparent from the subsequent description and the appended claims.

Definitions:

The pharmaceutically acceptable salts of the above tricyclics are an additional aspect of the invention. In those instances where the compounds of the invention possess acidic functional groups various salts may be formed which are more water soluble and physiologically suitable than the parent compound. Representative pharmaceutically acceptable salts include but are not limited to the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, *et al.,* "Pharmaceutical Salts," J. Phar. Sci., 66: 1-19 (1977)).

Compounds of the invention may have chiral centers and exist in optically active forms. R- and S-isomers and racemic mixtures are contemplated by this invention. A particular stereoisomer may be prepared by known methods using stereospecific reactions with starting materials containing asymmetric centers already resolved or, alternatively, by subsequent resolution of mixtures of stereoisomers using known methods.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include esters prepared by reaction of the parent acidic compound with a suitable alcohol. Simple aliphatic esters (methyl, ethyl, propyl, isopropyl, butyl, sec-butyl, tert-butyl) derived from acidic groups pendent on the compounds of this invention are preferred prodrugs. Other preferred esters include morpholinoethyloxy, diethylglycolamide and diethylaminocarbonylmethoxy.

The following list of abreviations are used in the Examples and Preparations.

| | |
|---|---|
| HCl = | hydrochloric acid |
| EtOAc = | ethyl acetate |
| DMF = | dimethyl formamide |
| THF = | tetrahydrofuran |
| $Et_2O$ = | diethyl ether |
| $H_2O$ = | water |
| NaOH = | sodium hydroxide |

| | |
|---|---|
| EtOH = | ethanol |
| $Na_2SO_4$ = | sodium sulfate |
| $NaHCO_3$ = | sodium bicarbonate |
| celite = | diatomaceous earth |
| $CH_2Cl_2$ = | methylene chloride |
| $H_2SO_4$ = | sulfuric acid |
| MeoH = | methanol |
| $Rh/Al_2O_3$ = | rhodium on alumina |
| DDQ = | 2,3-dichloro-5,6-dicyano-1,9-benzoquinone |
| TLC = | thin layer chromotography |
| NaH = | sodium hydride |
| $NH_4OH$ = | ammonium hydroxide |
| LiOH = | lithium hydroxide |
| $NH_3$ = | ammonia |
| $Cs_2CO_3$ = | cesium carbonate |
| $NH_4oAc$ = | ammonium acetate |

The following preparations of intermediates and examples of final products futher illustrate the preparation of the compounds of this invention. The examples are illustrative only and are not intended to limit the scope of the invention in any way.

Preparation 1

Preparation of 5-Carbomethoxy-1,2-dihydro-9H-carbazol-4(3H)-one from 2-bromo-3-nitrobenzoic acid

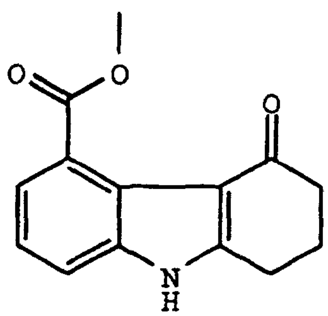

a) Methyl 2-bromo-3-nitrobenzoate

A solution of 2-bromo-3-nitrobenzoic acid (28.4 g, 115.0 mM), iodomethane (18.0 g, 127 mM), and potassium carbonate (19.0 g, 137.4 mM) in 100 mL DMF was stirred at room temperature for 72 hours. The mixture was poured into 1.5 liters of $H_2O$. The resultant precipitate was collected by filtration and dried *in vacuo* to afford 28.79 g (96%) of methyl 2-bromo-3-nitrobenzoate as a white solid. $^1H$ NMR (DMSO-d6) δ 8.3 (dd, 1H, J=1 and 8 Hz), 7.9 (dd, 1H, J=1 and 8 Hz), 7.7 (t, 1H, J=8 Hz), and 3.9 (s, 3H). IR (KBr, $cm^{-1}$) 2950, 1738, 1541, 1435, 1364, 1298, and 1142. MS (FD) m/e 259, 261.

| Elemental Analyses for $C_8H_6NO_4Br$: | | | |
|---|---|---|---|
| Calculated | C, 36.95; | H, 2.33; | N, 5.39. |
| Found | C, 37.14; | H, 2.37; | N, 5.45. |

b) Methyl 2-bromo-3-aminobenzoate

Hydrogen gas was passed through a solution of methyl 2-bromo-3-nitrobenzoate (0.20 g, 0.77 mM) and 0.1 g of 3% sulfided platinum on carbon in 25 mL ethyl acetate for 24 hours at room temperature. The catalyst was removed by filtration through celite. Concentration of the filtrate afforded 0.175 g (99%) of methyl 2-bromo-3-aminobenzoate as a yellow oil. $^1H$ NMR ($CDCl_3$) δ 7.15 (t, 1H, J=8 Hz) , 7.1 (dd, 1H, J=1 and 8 Hz), 6.8 (dd, 1H, J=1 and 8 Hz), and 3.95 (s, 3H). IR ($CHCl_3$, $cm^{-1}$) 3550, 3380, 2980, 2900, 1729, 1613, 1465, 1451, 1434, 1324, 1266,

and 1025. MS (FD) m/e 230, 232.

| Elemental Analyses for $C_8H_8NO_2Br$: | | | |
|---|---|---|---|
| Calculated | C, 41.77; | H, 3.51; | N, 6.09. |
| Found | C, 42.01; | H, 3.29; | N, 6.00. |

b)' In an alternate procedure, Methyl 2-bromo-3-aminobenzoate may be prepared as follows:

A solution of stannous chloride (15.0 g, 76.1 mM) in 30 mL of concentrated hydrochloric acid was slowly added to a solution of methyl 2-bromo-3-nitrobenzoate (4.0 g, 15.4 mM) in 90 mL ethanol at 15-30 °C over 1 hour. The mixture was then heated at 50-60 °C for 15 minutes. The mixture was cooled to room temperature and made alkaline by slow addition of solid sodium hydroxide maintaining a temperature of 30-35 °C. The resultant mixture was extracted three times with chloroform. The extracts were washed with brine, dried over sodium sulfate, filtered and concentrated to afford 3.51 g (99%) of methyl 2-bromo-3-aminobenzoate as a yellow oil, identical in all respects to the material derived via catalytic hydrogenation described above.

c) 3-(3-Carbomethoxy-2-bromoanilino)cyclohex-2-en-1-one

A mixture of methyl 2-bromo-3-aminobenzoate (13.2 g, 60.0 mM) and 1,3-cyclohexanedione (8.4 g, 75 mM) was heated at 125 °C under a stream of nitrogen for 4 h. The resultant solid was purified by HPLC on silica gel (elution with methylene chloride/ethyl acetate) to afford 17.2 g (88%) of 3-(3-carbomethoxy-2-bromoanilino)cyclohex-2-en-1-one as a tan foam. $^1H$ NMR (DMSO-d6) δ 8.75 (s, 1H), 7.6-7.4 (m, 3H), 4.65 (s, 1H), 3.85 (s, 3H), 2.6 (t, 2H, J=6 Hz), 2.15 (t, 2H, J=6 Hz), and 1.9 (m, 2H). IR ($CHCl_3$, $cm^{-1}$) 3400, 3004, 2954, 1732, 1607, 1588, 1573, 1513, 1464, 1436, 1412, 1308, 1249, 1177, and 1144. MS (ES) m/e 322, 324, 326.

| Elemental Analyses for $C_{14}H_{14}NO_3Br$: | | | |
|---|---|---|---|
| Calculated | C, 51.85; | H, 4.32; | N, 4.32. |
| Found | C, 53.60; | H, 4.73; | N, 4.09. |

d) 5-Carbomethoxy-1,2-dihydro-9H-carbazol-4(3H)-one

A suspension of 3-(3-carbomethoxy-2-bromoanilino)cyclohex-2-en-1-one (15.8 g, 48.8 mM), palladium acetate (1.12 g, 5.0 mM), tri-o-tolylphosphine (3.1 g, 10.0 mM), and triethylamine (6.3 g, 62.0 mM) in 120 mL acetonitrile was heated at reflux for 8 hours. The solvent was removed *in vacuo*. The residue was dissolved in methylene chloride, washed twice with 1 N HCl, twice with $H_2O$, once with saturated brine, dried over anhydrous magnesium sulfate, filtered, and concentrated to afford 17 g of a light brown foam. Purification by HPLC on silica gel (elution with gradient methylene chloride/ethyl acetate) afforded 9.2 g (78%) of the 5-carbomethoxy-1,2-dihydro-9H-carbazol-4(3H)-one as a yellow solid, identical with the material derived from 3-(3-carbomethoxy-2-chloroanilino)cyclohex-2-en-1-one, described above. $^1H$ NMR (DMSO-d6) δ 7.5 (d, 1H, J=8 Hz), 7.25-7.1 (m, 2H), 5.7 (s, 1H) , 3.8 (s, 3H), 2.95 (t, 2H, J=6 Hz), 2.4 (t, 2H, J=6 Hz), and 2.1 (m , 2H). MS (ES) m/e 242, 244.

EXAMPLE 1

Preparation of {9-[(phenyl)methyl]-5-carbamoylcarbazol-4-yl}oxyacetic acid, sodium salt

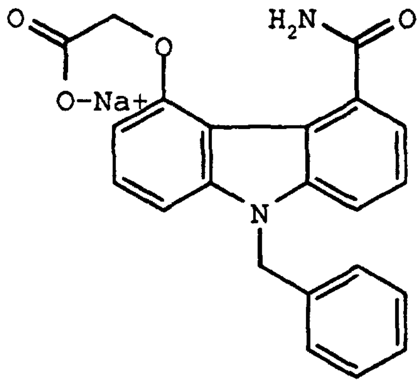

A. 9-[(Phenyl)methyl]-5-carbomethoxy-1,2-dihydrocarbazol-4(3H)-one

A suspension of 5-carbomethoxy-1,2-dihydro-9H-carbazol-4(3H)-one (300 mg, 1.23 mM), benzyl bromide (210 mg, 1.23 mM), and potassium carbonate (170 mg, 1.23 mM) in 15 mL DMF was stirred at room temperature for 6 hours. The mixture was diluted with 80 mL $H_2O$ and chilled in the refrigerator. The resultant white precipitate was collected by filtration, washed with $H_2O$, and dried *in vacuo* to afford 325 mg (79%) of the 9-[(phenyl)methyl]-5-carbomethoxy-1,2-dihydrocarbazol-4(3H)-one as a white solid. $^1H$ NMR (DMSO-d6) δ 7.7 (dd, 1H, J=1 and 8 Hz), 7.45-7.0 (m, 7H), 5.6 (s, 2H), 3.8 (s, 3H), 3.05 (t, 2H, J=6 Hz), 2.5 (t, 2H, J=6 Hz), and 2.2 (m , 2H). IR (KBr, $cm^{-1}$) 3421, 1726, 1676, 1636, 1473, 1450, 1435, 1288, 1122, 764, 745, and 706. MS (ES) m/e 334.

| Elemental Analyses for $C_{21}H_{19}NO_3$: | | | |
|---|---|---|---|
| Calculated | C, 75.68; | H, 5.71; | N, 4.20. |
| Found | C, 70.85; | H, 5.53; | N, 4.49. |

B. 9-[(Phenyl)methyl]-4-hydroxy-5-carbomethoxy carbazole (a) A solution of the 9-[(phenyl)methyl]-5-carbomethoxy-1,2-dihydrocarbazol-4(3H)-one (1.5 g, 4.5 mM) and 2,3-dichloro-5,6-dicyano-1,4-benzoquinone (1.12 g, 5.0 mM) in 25 mL of toluene was stirred between 80-90 °C for 6 h. The mixture was purified directly by column chromatography on silica gel (elution with methylene chloride/ethyl acetate) to afford 420 mg (28%) of the 9-[(phenyl) methyl]-4-hydroxy-5-carbomethoxy carbazole as a yellow solid. $^1H$ NMR (DMSO-d6) δ 10.25 (s, 1H), 7.7 (d, 1H, J=8 Hz), 7.4 (t, 1H, J=8 Hz), 7.4-7.0 (m, 8H), 6.6 (d, 1H, J=8 Hz), 5.6 (s, 2H), and 3.8 (s, 3H). IR ($CHCl_3$, $cm^{-1}$) 1723, 1685, 1621, 1597, 1568, 1496, 1453, 1442, 1392, 1286, 1267, 1156, and 1138. MS (ES) m/e 330, 332.

| Elemental Analyses for $C_{21}H_{17}NO_3$: | | | |
|---|---|---|---|
| Calculated | C, 76.13; | H, 5.14; | N, 4.23. |
| Found | C, 75.90; | H, 5.20; | N, 4.46. |

(b) To a solution of the 9-[(phenyl)methyl)-5-carbomethoxy-1,2-dihydrocarbazol-4(3H)-one (2.87 g, 8.61 mM) in 29 ml dioxane was added 60% sodium hydride in mineral oil (0.79 g, 19.8 mM). The reaction was stirred 8 minutes, then methyl benzenesulfinate (1.80 ml, 13.8 mM) was added. The reaction was stirred an additional 1.5 h, then diluted with 43 ml dioxane and 1.13 ml acetic acid. The mixture was refluxed 1 h, diluted with ethyl acetate, and extracted with sat'd $NaHCO_3$ two times, then with brine. After drying ($NaSO_4$), evaporation in vacuo afforded 4.90 g. The mixture was purified by column chromatography on silica gel (elution with toluene/methylene chloride) to afford 2.31 g (81%) of the 9-[(phenyl)methyl]-4-hydroxy-5-carbomethoxy carbazole. $^1H$ NMR (DMSO-d6) δ 10.25 (s, 1H), 7.7 (d, 1H, J=8 Hz), 7.4 (t, 1H, J=8 Hz), 7.4-7.0 (m, 8H), 6.6 (d, 1H, J=8 Hz), 5.6 (s, 2H), and 3.8 (s, 3H). IR ($CHCl_3$, $cm^{-1}$) 1723, 1685, 1621, 1597, 1568, 1496, 1453, 1442, 1392, 1286, 1267, 1156, and 1138. MS (ES) m/e 330, 332.

| Elemental Analyses for $C_{21}H_{17}NO_3$: | | | |
|---|---|---|---|
| Calculated | C, 76.13; | H, 5.14; | N, 4.23. |
| Found | C, 75.90; | H, 5.20; | N, 4.46. |

C. 9-[(Phenyl)methyl]-4-hydroxy-5-carbamoyl carbazole

A solution of the 9-[(phenyl)methyl]-4-hydroxy-5-carbomethoxy carbazole (200 mg, 0.6 mM) in 4 mL MeOH and 40 mL concentrated aqueous ammonium hydroxide was sonicated for 30 h at 40-50 °C. The mixture was diluted with ethyl acetate and acidified to pH 1 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 50 mg (26%) of the 9-[(phenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.85 (dd, 1H, J=1 and 8 Hz), 7.5-7.1 (m, 9H), 6.6 (d, 1H, J=8 Hz), and 5.8 (s, 2H). IR (KBr, $cm^{-1}$) 3428, 3198, 3063, 1631, 1599, 1579, 1562, 1496, 1442, 1330, 1261, 1215, 775, and 697. MS (ES) m/e 315, 317.

| Elemental Analyses for $C_{20}H_{16}N_2O_2$: | | | |
|---|---|---|---|
| Calculated | C, 75.95; | H, 5.06; | N, 8.86. |
| Found | C, 74.88; | H, 5.40; | N, 7.78. |

D. {9-[(Phenyl)methyl]-5-carbamoylcarbazol-4-yl}oxyacetic acid, methyl ester

40% Methanolic Triton B (0.11 mL, 0.24 mM) was added to a solution of the 9-[(phenyl)methyl]-4-hydroxy-5-carbamoyl carbazole (70 mg, 0.22 mM) in 20 mL DMF at 0 °C. After 15 minutes, methyl bromoacetate (70 mg, 0.44 mM) was added and the resultant mixture stirred at room temperature for 5 h. The mixture was diluted with ethyl acetate, washed with 1 N HCl, $H_2O$, and saturated brine, dried over magnesium sulfate, filtered, and concentrated. The residue was combined with the crude material derived from a similar run utilizing 45 mg (0.14 mM [0.36 mM total]) of 9-[(phenyl)methyl]-4-hydroxy-5-carbamoyl carbazole. The combined residues were purified by column chromatography on silica gel (elution with ethyl acetate) to afford 76 mg (54%) of the {9-[(phenyl)methyl]-5-carbamoylcarbazol-4-yl}oxyacetic acid, methyl ester as a white solid. $^1H$ NMR (DMSO-d6) δ 7.65 (d, 1H, J=8 Hz), 7.5 (br s, 1H), 7.4-7.15 (m, 9H), 7.1 (d, 1H, J=8 Hz), 6.6 (d, 1H, J=8 Hz), 5.7 (s, 2H), 4.9 (s, 2H), and 3.75 (s, 3H). IR (KBr, $cm^{-1}$) 3367, 3200, 1760, 1643, 1579, 1496, 1452, 1427, 1216, 1157, 772, and 716. MS (FD) m/e 388.

| Elemental Analyses for $C_{23}H_{20}N_2O_4$: | | | |
|---|---|---|---|
| Calculated | C, 71.13; | H, 5.15; | N, 7.22. |
| Found | C, 70.77; | H, 5.49; | N, 6.79. |

E. {9-[(Phenyl)methyl]-5-carbamoylcarbazol-4-yl}oxyacetic acid, sodium salt.

A solution of the {9-[(phenyl)methyl]-5-carbamoylcarbazol-4-yl}oxyacetic acid, methyl ester (10.1 mg, 0.025 mM) and 0.025 mL (0.025 mM) of 1 N NaOH in 3 mL of ethanol was stirred for 16 h at 25 °C. The resultant white precipitate was collected by filtration, washed with a small amount of EtOH, then dried *in vacuo* to afford 7.1 mg (70%) of the {9-[(phenyl)methyl]-5-carbamoylcarbazol-4-yl}oxyacetic acid, sodium salt as a white powder. $^1H$ NMR δ 7.6 (d, 1H, J=8 Hz), 7.5-7.05 (m, 11H), 6.55 (d, 1H, J=8 Hz), 5.75 (s, 2H), and 4.3 (s, 2H). IR (KBr, $cm^{-1}$) 3471, 1657, 1615, 1591, 1496, 1453, 1412, 1330, 1272, and 1151. MS (ES) m/e 373, 375, 397. Elemental Analyses for $C_{22}H_{17}N_2O_4Na$: C, 66.67; H, 4.29; N, 7.07. Found C, 66.75; H, 4.55; N, 6.83.

Example 2

Preparation of [9-benzyl-4-carbamoyl-8-fluoro-1,2,3,4-tetrahydrocarbazol-5-yl]oxyacetic acid

A. Preparation of (2-chloro-4-fluorophenyl)- ethyl carbonate

A solution of 19.16 g of 2-chloro-4-fluorophenol in 65.4 ml of 2 N aqueous sodium hydroxide solution was cooled in an ice bath and treated dropwise with 16.3 ml of ethyl chloroformate. After stirring at room temperature overnight, the two-phase reaction mixture was diluted with 100 ml of water and extracted with 300 ml of a 1:1

pentane/ether mixture. The extract was washed three times with 0.02 N sodium hydroxide solution, water and then brine. After drying and evaporation, 27.63 g (97%) of the subtitle compound were obtained. NMR (300 MHz, $CDCl_3$): δ 7.23-7.18 (m, 2H); 7.00 (dt, J=8.4, 2.7, 1H); 4.35 (q, J=7.1, 2H); 1.40 (t, J=7.1, 3H).

B. Preparation of (2-chloro-4-fluoro-5-nitrophenyl)- ethyl carbonate

A solution of 27.63 g of (2-chloro-4-fluorophenyl)-ethyl carbonate in 60 ml of dichloromethane was cooled in an ice bath and treated dropwise with 31.86 g of a 1:2 mixture of fuming nitric acid (90%) and concentrated sulfuric acid. The reaction was stirred for 2 hours at room temperature and then cooled with ice and treated with another 4.5 g of the same nitrating mixture. The reaction was stirred overnight at room temperature, poured into 200 ml of ice and water,and extracted twice with dichloromethane. The extracts were washed with water and then with brine, dried over magnesium sulfate and concentrated to afford 33.01 g (99%) of the subtitle compound. mp. 50-51 C

| Elemental Analyses | | | | |
|---|---|---|---|---|
| Calculated | C 41.01; | H 2.68; | N 5.31; | Cl 13.45 |
| Found | C 41.03; | H 2.59; | N 5.38; | Cl 13.71 |

C. Preparation of 2-chloro-4-fluoro-5-nitroanisole

A solution of 15.0 g of (2-chloro-4-fluoro-5-nitrophenyl)- ethyl carbonate in 100 ml of dimethyl formamide was treated with 18.6 g of cesium carbonate, 7.1 ml of iodomethane and 7 ml of methanol and stirred overnight at room temperature. The reaction mixture was poured into water and extracted twice with ether. The extracts were washed twice with water and then with brine, dried over magnesium sulfate and concentrated to afford 11.4g of the subtitle compound. mp. 69-70°C Ex. 57, C.

| Elemental Analyses | | | | |
|---|---|---|---|---|
| Calculated | C 40.90; | H 2.45; | N 6.81; | Cl 17.25 |
| Found | C 41.20; | H 2.48; | N 6.70; | Cl 17.44 |

D. Preparation of 2-fluoro-5-methoxyaniline

A solution of 5.63 g of 2-chloro-4-fluoro-5-nitroanisole in 90 ml of ethanol and 5 ml of triethylamine was hydrogenated at room temperature under 60 pounds per square inch with 1.0 g of 5% palladium on carbon for four hours. The catalyst was filtered off and the solvent was evaporated. The residue was slurried in chloroform and filtered thourough a plug of silica gel and then evaporated. This residue was chromatographed on silica gel using hexane/chloroform mixtures to afford 2.77 g (72%) of the subtitle compound. mp. 253-254°C. NMR (300 MHz, $CDCl_3$): δ 6.88 (dd, J=10.6, 8.9, 1H); 6.32 (dd, J=7.4, 3.0, 1H); 6.20 (dt, J=8.9, 3.2,1H); 3.73 (s, 3H); 3.72 (br, 2H).

E. Preparation of N-benzyl-2-fluoro-5-methoxyaniline

This procedure was patterned after that of Tietze and Grote, Chem Ber. 126(12), 2733 (1993). A solution of 2.73 g of 2-fluoro-5-methoxyaniline and 2.67 g of benzaldehyde in 48 ml of methanol was treated with 3.43 g of zinc chloride and then cooled in an ice bath. Sodium cyanoborohydride (1.58 g) was added in small poroom temperature ions over 30 minutes and the reaction was stirred for five hours at room temperature. After evaporation of the solvent, the residue was slurried in 40 ml of 1 N sodium hydroxide solution and then extracted twice with ether. The extracts were washed with water and then with brine, dried over magnesium sulfate and concentrated. The residue was recrystallized from hexane to afford 2.61 g and the mother liquors were chromatographed on silica gel using 20:1 hexane/ether to afford another 1.4 g of the subtitle compound (90%). mp. 56-58°C

| Elemental Analyses | | | |
|---|---|---|---|
| Calculated | C 72.71; | H 6.10; | N 6.06 |
| Found | C 72.51; | H 6.06; | N 5.99 |

F. Preparation of ethyl 9-benzyl-5-methoxy-8-fluoro-1,2,3,4-tetrahydrocarbazole-4-carboxylate

A solution of 0.62 g of N-benzyl-2-fluoro-5-methoxyaniline in 20 ml of dry tetrahydrofuran was cooled in an ice bath and treated with 11.3 ml of 0.5 M potassium bis(trimethylsilyl)amide in toluene. After stirring for 30 minutes, 0.74 g of 2-carboethoxy-6-bromocyclohexanone (Sheehan and Mumaw, JACS, 72, 2127 (1950)) in 4 ml of tetrahydrofuran was added and the reaction was allowed to warm slowly to room temperature over two hours. The reaction was quenched with saturated ammonium chloride solution and extracted twice with ether. The extracts

were washed with water and then with brine, dried over magnesium sulfate and concentrated. This residue was chromatographed on silica gel using hexane/ ether mixtures to afford 0.796 g (74%) of N-alkylated intermediate diastereomers. This mixture was refluxed in 20 ml of benzene with 0.99 g of zinc chloride overnight. The solvent was evaporated and the residue was partitioned between 25 ml of 1 N HCl and 25 ml of ethyl acetate and then extracted once more with ethyl acetate. The organic layers were washed with water and then brine, dried over magnesium sulfate and concentrated to afford 0.734 g (96%) of the subtitled compound. ESIMS *m/e* 382 ($M^+$+1)

| Elemental Analyses | | | |
|---|---|---|---|
| Calculated | C 72.42; | H 6.34; | N 3.67 |
| Found | C 72.20; | H 6.26; | N 3.70 |

G. Preparation of 9-benzyl-5-methoxy-8-fluoro-1,2,3,4-tetrahydrocarbazole-4-carboxamide

Ethyl 9-benzyl-5-methoxy-8-fluoro-1,2,3,4-tetrahydrocarbazole-4-carboxylate (0.722 g) was treated similarly as described in Example 49, Part C and chromatographed on silica gel using 1% methanol in dichloromethane to afford 0.482 g (72%) of the subtitle compound. ESIMS *m/e* 353 ($M^+$+1)

| Elemental Analyses | | | |
|---|---|---|---|
| Calculated | C 71.57; | H 6.01; | N 7.95 |
| Found | C 71.42; | H 5.83; | N 7.75 |

H. Preparation of [9-benzyl-4-carbamoyl-8-fluoro-1,2,3,4-tetrahydrocarbazol-5-yl]oxyacetic acid methyl ester

9-Benzyl-5-methoxy-8-fluoro-1,2,3,4-tetrahydrocarbazole-4-carboxamide (0.170 g) was converted similarly as described in Example 49, Part D and chromatographed on silica gel using methanol/ 0-1% in dichloromethane to afford 85 mg (50%) of the subtitle compound. mp. 183-185°C

| Elemental Analyses | | | |
|---|---|---|---|
| Calculated | C 67.31; | H 5.65; | N 6.82 |
| Found | C 67.58; | H 5.48; | N 6.95 |

I. Preparation of [9-benzyl-4-carbamoyl-8-fluoro-1,2,3,4-tetrahydrocarbazol-5-yl]oxyacetic acid

[9-Benzyl-4-carbamoyl-8-fluoro-1,2,3,4-tetrahydrocarbazol-5-yl]oxyacetic acid methyl ester (71 mg) was hydrolyzed similarly as described in Example 50, Part D to afford 65 mg of the title compound. ESIMS m/e 397 ($M^+$+1), 395 ($M^+$-1) NMR (300 MHz, $d^{6-}$DMSO): δ 13.03 (br, 1H); 7.31-7.19 (m, 3H); 6.97 (d, J=7.4, 2H); 6.95 (br, 1H); 6.70 (d, J=3.8, 1H); 6.67 (dd, J=12.4, 3.9, 1H); 6.28 (dd, J=8.5, 2.6, 1H); 5.39 (ABq, 2H); 4.64 (s, 2H); 3.92 (br, 1H); 2.71 (m, 1H); 2.44 (m, 1H); 2.02 (m, 2H); 1.76 (m, 2H).

Example 3

Preparation of [9-benzyl-5-carbamoyl-1-fluorocarbazol-4-yl]oxyacetic acid

A. Preparation of 9-benzyl-5-carbamoyl-4-methoxy-1-fluorocarbazole

A solution of 0.458 g of 9-benzyl-5-methoxy-8-fluoro-1,2,3,4-tetrahydrocarbazole-4-carboxamide in 13 ml of dry dioxane under nitrogen was treated with 0.59 g of 2,3-dichloro-5,6-dicyano-1,4-benzoquinone and refluxed for one hour. The reaction mixture was cooled and filtered and the precipitate was washed with 15 ml of dioxane. The filtrate and washing were poured into saturated sodium bicarbonate solution and extracted three times with ethyl acetate. The extracts were washed with saturated sodium bicarbonate, with water and then with brine; dried over magnesium sulfate and concentrated. This residue was chromatographed on silica gel using dichloromethane/ 0-2% methanol to afford 0.45 g of subtitle compound. ESIMS m/e 349 ($M^+$+1)

| Elemental Analyses | | | |
|---|---|---|---|
| Calculated | C 72.42; | H 4.92; | N 8.04 |
| Found | C 72.35; | H 4.81; | N 7.88 |

B. Preparation of [9-benzyl-5-carbamoyl-l-fluorocarbazol-4-yl]oxyacetic acid methyl ester

A solution of 0.45 g of 9-benzyl-5-carbamoyl-4-methoxy-1-fluorocarbazole in 25 ml of dichloromethane was cooled in an ice bath treated dropwise with 12 ml of 1.0 M boron tribromide solution in dichloromethane. The reaction was allowed to warm to room temperature slowly over 2 hours and then quenched by pouring into ice and then adding 50 ml of 1 N HCl. The mixture was extracted with dichloromethane (3x200 ml) and the extracts were dried over magnesium sulfate and concentrated to afford 0.35 g (78%) of the demethylated intermediate. This intermediate (0.215 g) was alkylated and purified similarly to Example GH1, Part D to afford 0.166 g (64%) of the subtitle compound. mp. 190-191°C

| Elemental Analyses | | | |
|---|---|---|---|
| Calculated | C 67.97; | H 4.71; | N 6.89 |
| Found | C 67.81; | H 4.94; | N 6.96 |

C. Preparation of [9-benzyl-5-carbamoyl-1-fluorocarbazol-4-yl]oxyacetic acid

[9-Benzyl-5-carbamoyl-l-fluorocarbazol-4-yl]oxyacetic acid methyl ester (56 mg) was hydrolyzed and isolated similarly as described in Example 50, Part D to afford 54 mg of the title compound. FDMS *m*/*e* 392 ($M^+$); ESIMS *m*/*e* 393 ($M^+$+1), 391 ($M^+$-1) NMR (300 MHz, $d^6$-DMSO): δ 12.92 (br, 1H); 7.70 (m, 2H); 7.45 (t, J=7.5, 1H); 7.39 (br, 1H); 7.28-7.17 (m, 4H); 7.12 (d, J=7.2, 1H); 7.07 (d, J=7.0, 2H); 6.51 (dd, J=8.8, 2.7, 1H); 5.77 (s, 2H); 4.80 (s, 2H).

| Elemental Analyses | | | |
|---|---|---|---|
| Calculated | C 67.34; | H 4.37; | N 7.14 |
| Found | C 66.92; | H 4.49; | N 6.77 |

EXAMPLE 4

Preparation of {9-[(3-fluorophenyl)methyl]-5-carbamoylcarbazol-4-yl}oxyacetic acid

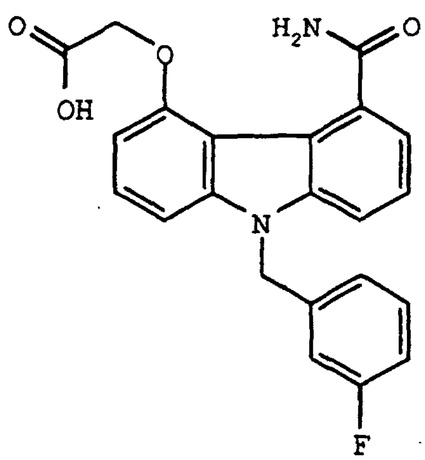

A. 9-[(3-Fluorophenyl)methyl]-5-carbomethoxy-1,2-dihydrocarbazol-4(3H)-one

40% Methanolic Triton B (2.06 mL, 4.53 mM) was slowly added dropwise to a solution of 5-carbomethoxy-1,2-dihydro-9H-carbazol-4(3H)-one (930.0 mg, 3.82 mM) in 5 mL of DMF at 0 °C. After 5 minutes, 3-fluorobenzyl chloride (664.0 mg, 4.59 mM) was added and the resultant mixture stirred at 0 °C for 3 h, then at room temperature for 20 hours. The mixture was diluted with ethyl acetate, washed three times with 1 N HCl, three times with $H_2O$, once with saturated brine, dried over anhydrous magnesium sulfate, filtered, and concentrated. The residue was purified by column chromatography on silica gel (elution with gradient methylene chloride/ethyl acetate) to afford 502.3 mg (37%) of the 9-[(3-fluorophenyl)methyl]-5-carbomethoxy-1,2-dihydrocarbazol-4(3H)-one as a yellow

foam. $^1H$ NMR ($CDCl_3$) δ 7.4-7.2 (m, 4H), 6.9 (m, 1H), 6.7 (m, 2H), 5.35 (s, 2H), 4.05 (s, 3H), 2.9 (t, 2H, J=6 Hz), 2.65 (t, 2H, J=6 Hz), and 2.3 (m , 2H). IR ($CHCl_3$, $cm^{-1}$) 3050, 2950, 1725, 1654, 1464, 1451, 1440, 1288 and 1119. MS (ES) m/e 350, 352.

| Elemental Analyses for $C_{21}H_{18}NO_3F$: | | | |
|---|---|---|---|
| Calculated | C, 71.78; | H, 5.16; | N, 3.99. |
| Found | C, 72.00; | H, 4.95; | N, 4.11. |

B. 9-[(3-Fluorophenyl)methyl]-4-hydroxy-5-carbomethoxy carbazole

A solution of the 9-[(3-fluorophenyl)methyl]-5-carbomethoxy-1,2-dihydrocarbazol-4 (3H)-one (434.0 mg, 1.23 mM) and 2,3-dichloro-5,6-dicyano-1,4-benzoquinone (324.0 mg, 1.42 mM) in 20 mL of toluene was stirred between 70-80 °C for 5 h. The mixture was purified directly by column chromatography on silica gel (elution with methylene chloride) to afford 137.0 mg (32%) of the 9-[(3-fluorophenyl)methyl]-4-hydroxy-5-carbomethoxy carbazole as a yellow foam. $^1H$ NMR (DMSO-d6) δ 10.2 (s, 1H), 7.7 (d, 1H, J=8 Hz), 7.4 (t, 1H, J=8 Hz), 7.3 (m, 2H), 7.2 (d, 1H, J=8 Hz), 7.1 (d, 1H, J=8 Hz), 7.05-6.85 (m, 3H), 6.6 (d, 1H, J=8 Hz), 5.65 (s, 2H), and 3.85 (s, 3H). IR ($CHCl_3$, $cm^{-1}$) 3200 (br), 1687, 1597, 1452, 1442, 1285, and 1267. MS (ES) m/e 348, 350.

| Elemental Analyses for $C_{21}H_{16}NO_3F$: | | | |
|---|---|---|---|
| Calculated | C, 72.20; | H, 4.62; | N, 4.01. |
| Found | C, 72.30; | H, 4.66; | N, 4.04. |

C. 9-[(3-Fluorophenyl)methyl]-4-hydroxy-5-carbamoyl carbazole

A solution of the 9-[(3-fluorophenyl)methyl]-4-hydroxy-5-carbomethoxy carbazole (130.8 mg, 0.37 mM) in 5 mL THF and 20 mL concentrated aqueous ammonium hydroxide was sonicated for 5 h at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 57.4 mg (45%) of the 9-[(3-fluorophenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.8 (dd, 1H, J=1 and 8 Hz), 7.5 (m, 2H), 7.3 (m, 2H), 7.15-7.0 (m, 2H), 6.95 (d, 1H, J=8 Hz), 6.85 (d, 1H, J=8 Hz), 6.6 (d, 1H, J=8 Hz), and 5.7 (s, 2H). IR ($CHCl_3$, $cm^{-1}$) 3431, 3200 (br), 1628, 1614, 1600, 1580, 1546, 1488, 1448, 1329, 1261, and 776. MS (ES) m/e 333, 335.

| Elemental Analyses for $C_{20}H_{15}N_2O_2F$: | | | |
|---|---|---|---|
| Calculated | C, 71.85; | H, 4.52; | N, 8.38. |
| Found | C, 74.45; | H, 6.01; | N, 8.48. |

D. {9-[(3-Fluorophenyl)methyl]-5-carbamoylcarbazol-4-yl}oxyacetic acid, tert-butyl ester

40% Methanolic Triton B (0.086 mL, 0.19 mM) was added to a solution of the 9-[(3-fluorophenyl)methyl]-4-hydroxy-5-carbamoyl carbazole (51.9 mg, 0.155 mM) in 3 mL DMF at room temperature. After 3 minutes, t-butyl bromoacetate (87.8 mg, 0.44 mM) was added and the resultant mixture stirred at room temperature for 5 hours. The mixture was diluted with ethyl acetate, washed four times with $H_2O$, and saturated brine, dried over magnesium sulfate, filtered, and concentrated. The residue was purified by column chromatography on silica gel (elution with gradient methylene chloride/ethyl acetate) to afford 44.0 mg (63%) of the {9-[(3-fluorophenyl)methyl]-5-carbamoylcarbazol-4-yl}oxyacetic acid, tert-butyl ester as a white solid. $^1H$ NMR (DMSO-d6) δ 7.6 (d, 1H, J=8 Hz), 7.5-6.8 (m, 10H), 6.55 (d, 1H, J=8 Hz), 5.7 (s, 2H), 4.8 (s, 2H), and 1.45 (s, 9H). IR ($CHCl_3$, $cm^{-1}$) 3450, 3400, 1746, 1674, 1592, 1457, 1369, and 1151. MS (FD) m/e 448.

| Elemental Analyses for $C_{26}H_{25}N_2O_4F$: | | | |
|---|---|---|---|
| Calculated | C, 69.63; | H, 5.62; | N, 6.25. |
| Found | C, 69.35; | H, 5.44; | N, 6.23. |

E. {9-[(3-Fluorophenyl)methyl]-5-carbamoylcarbazol-4-yl}oxyacetic acid

A solution of the {9-[(3-fluorophenyl)methyl]-5-carbamoylcarbazol-4-yl}oxyacetic acid, tert-butyl ester (40.0

mg, 0.089 mM) in 2 mL of trifluoroacetic acid was stirred at room temperature for 5 hours. The solvent was removed *in vacuo.* The residue was triturated with ethyl ether, then dried *in vacuo* to afford 35.0 mg (100%) of the {9-[(3-fluorophenyl)methyl]-5-carbamoylcarbazol-4-yl}oxyacetic acid as a white powder. $^1$H NMR (DMSO-d6) δ 13.0 (br s, 1H), 7.75 (s, 1H), 7.6 (d, 1H, J=8 Hz), 7.5-7.25 (m, 5H), 7.2-6.8 (m, 4H), 6.6 (d, 1H, J=8 Hz), 5.7 (s, 2H), and 4.8 (s, 2H). IR (KBr, $cm^{-1}$) 3423, 3400, 1736, 1637, 1615, 1589, 1499, 1487, 1450, 1436, 1331, 1250, and 1156. MS (ES) m/e 391, 393.

| Elemental Analyses for $C_{22}H_{17}N_2O_4F$: | | | |
|---|---|---|---|
| Calculated | C, 67.34; | H, 4.37; | N, 7.14. |
| Found | C, 67.63; | H, 4.22; | N, 7.35. |

EXAMPLE 5

Preparation of {9-[(3-Chlorophenyl)methyl]-5-carbamoylcarbazol-4-yl}oxyacetic acid

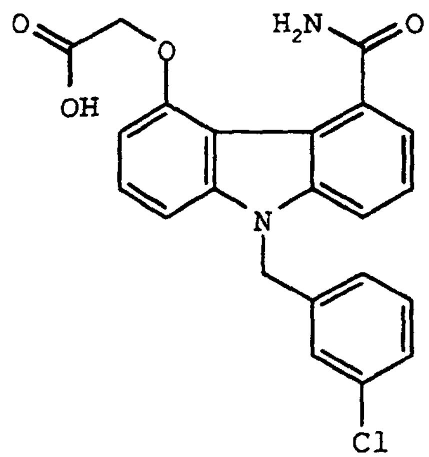

A. 9-[(3-Chlorophenyl)methyl]-5-carbomethoxy-1,2-dihydrocarbazol-4 (3H)-one

A suspension of 5-carbomethoxy-1,2-dihydro-9H-carbazol-4(3H)-one (527.0 mg, 2.17 mM), 3-chlorobenzyl bromide (802.2 mg, 3.90 mM), a catalytic amount of sodium iodide (ca. 1 mg), and potassium carbonate (500.0 mg, 3.62 mM) was stirred at room temperature for 150 hours. The mixture was diluted with ethyl acetate, washed five times with $H_2O$, once with saturated brine, dried over anhydrous magnesium sulfate, filtered, and concentrated. The residue was purified by column chromatography on silica gel (elution with gradient methylene chloride/ethyl acetate) to afford 537.1 mg (67%) of the 9-[(3-chlorophenyl)methyl]-5-carbomethoxy-1,2-dihydrocarbazol-4(3H)-one as a yellow foam. $^1$H NMR ($CDCl_3$) δ 7.5-7.2 (m, 5H), 7.1 (s, 1H), 6.85 (m, 1H), 5.35 (s, 2H), 4.05 (s, 3H), 2.9 (t, 2H, J=6 Hz), 2.65 (t, 2H, J=6 Hz), and 2.3 (m , 2H). IR ($CHCl_3$, $cm^{-1}$) 3050, 2950, 1725, 1654, 1464, 1444, 1432, 1288 and 1120. MS (ES) m/e 366, 368, 370.

| Elemental Analyses for $C_{21}H_{18}NO_3Cl$: | | | |
|---|---|---|---|
| Calculated | C, 68.57; | H, 4.93; | N, 3.81. |
| Found | C, 68.61; | H, 4.92; | N, 3.70. |

B. 9-[(3-Chlorophenyl)methyl]-4-hydroxy-5-carbomethoxy carbazole

A solution of the 9-[(3-chlorophenyl)methyl]-5-carbomethoxy-1,2-dihydrocarbazol-4(3H)-one (480.5 mg, 1.31 mM) and 2,3-dichloro-5,6-dicyano-1,4-benzoquinone (325.7 mg, 1.43 mM) in 50 mL of toluene was stirred between 70-80 °C for 3 hours. The mixture was purified directly by column chromatography on silica gel (elution with methylene chloride) to afford 172.6 mg (36%) of the 9-[(3-chlorophenyl)methyl]-4-hydroxy-5-carbomethoxy carbazole as a yellow foam. $^1$H NMR ($CDCl_3$) δ 10.4 (s, 1H), 8.05 (d, 1H, J=8 Hz), 7.6 (d, 1H, J=8 Hz), 7.4 (m, 2H), 7.3-7.1

(m, 3H), 6.9-6.7 (m, 3H), 5.55 (s, 2H), and 4.15 (s, 3H). IR ($CHCl_3$, $cm^{-1}$) 3200 (br), 1684, 1598, 1442, 1428, 1331, 1285, and 1267. MS (ES) m/e 364, 366, 368.

| Elemental Analyses for $C_{21}H_{16}NO_3Cl$: | | | |
|---|---|---|---|
| Calculated | C, 68.95; | H, 4.41; | N, 3.83. |
| Found | C, 69.23; | H, 4.52; | N, 3.88. |

C. 9-[(3-Chlorophenyl)methyl]-4-hydroxy-5-carbamoyl carbazole

A solution of the 9-[(3-chlorophenyl)methyl]-4-hydroxy-5-carbomethoxy carbazole (156.2 mg, 0.43 mM) in 5 mL THF and 20 mL concentrated aqueous ammonium hydroxide was sonicated for 5 hours at 40-50 °C. The mixture was diluted with ethyl acetate and acidified to pH 1 with 5 N HCl. The aqueous layer was extracted twice with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 69.7 mg (47%) of the 9-[(3-chlorophenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.8 (dd, 1H, J=1 and 8 Hz), 7.45 (m, 2H), 7.3 (m, 3H), 7.2 (s, 1H), 7.1 (d, 1H, J=8 Hz), 6.95 (s, 1H), 6.6 (d, 1H, J=8 Hz), and 5.7 (s, 2H). IR ($CHCl_3$, $cm^{-1}$) 3433, 3202 (br), 1630, 1600, 1580, 1564, 1433, 1330, 1261, and 776. MS (ES) m/e 349, 351, 353.

| Elemental Analyses for $C_{20}H_{15}N_2O_2Cl$: | | | |
|---|---|---|---|
| Calculated | C, 68.48; | H, 4.31; | N, 7.99. |
| Found | C, 68.64; | H, 4.55; | N, 7.93. |

D. {9-[(3-Chlorophenyl)methyl]-5-carbamoylcarbazol-4-yl}oxyacetic acid, tert-butyl ester

40% Methanolic Triton B (0.053 mL, 0.12 mM) was added to a solution of the 9-[(3-chlorophenyl)methyl]-4-hydroxy-5-carbamoyl carbazole (33.2 mg, 0.12 mM) in 2 mL DMF at room temperature. After 3 minutes, t-butyl bromoacetate (53.8 mg, 0.27 mM) was added and the resultant mixture stirred at room temperature for 20 h. The mixture was diluted with ethyl acetate, washed four times with $H_2O$, once with saturated brine, dried over magnesium sulfate, filtered, and concentrated. The residue was purified by column chromatography on silica gel (elution with gradient methylene chloride/ethyl acetate) to afford 42.1 mg (95%) of the {9-[(3-chlorophenyl)methyl]-5-carbamoylcarbazol-4-yl)oxyacetic acid, tert-butyl ester as a white solid. $^1H$ NMR (DMSO-d6) δ 7.6 (d, 1H, J=8 Hz), 7.5-6.8 (m, 10H), 6.55 (d, 1H, J=8 Hz), 5.7 (s, 2H), 4.8 (s, 2H), and 1.45 (s, 9H). IR ($CHCl_3$, $cm^{-1}$) 3450, 3400, 1744, 1676, 1591, 1457, 1369, and 1150. MS (FD) m/e 464, 466.

| Elemental Analyses for $C_{26}H_{25}N_2O_4Cl$: | | | |
|---|---|---|---|
| Calculated | C, 67.17; | H, 5.42; | N, 6.03. |
| Found | C, 67.17; | H, 5.65; | N, 5.97. |

E. {9-[(3-Chlorophenyl)methyl]-5-carbamoylcarbazol-4-yl}oxyacetic acid

A solution of the {9-[(3-chlorophenyl)methyl]-5-carbamoylcarbazol-4-yl}oxyacetic acid, tert-butyl ester (35.6 mg, 0.077 mM) in 2 mL of trifluoroacetic acid was stirred at room temperature for 6 hours. The solvent was removed *in vacuo.* The residue was triturated with ethyl acetate, then dried *in vacuo* to afford 31.4 mg (100%) of the {9-[(3-chlorophenyl)methyl]-5-carbamoylcarbazol-4-yl}oxyacetic acid as a white powder. $^1H$ NMR (DMSO-d6) δ 13.0 (br s, 1H), 7.75 (s, 1H), 7.6 (d, 1H, J=8 Hz), 7.4-7.25 (m, 7H), 7.2 (d, 1H, J=8 Hz), 7.0 (br t, 1H), 6.6 (d, 1H, J=8 Hz), 5.7 (s, 2H), and 4.8 (s, 2H). IR (KBr, $cm^{-1}$) 3456, 3416, 3335, 1735, 1638, 1617, 1580, 1499, 1452, 1431, 1431, 1329, 1255, 1157, 772, 764, and 717. MS (ES) m/e 407, 409, 411.

| Elemental Analyses for $C_{22}H_{17}N_2O_4Cl$: | | | |
|---|---|---|---|
| Calculated | C, 64.63; | H, 4.19; | N, 6.85. |
| Found | C, 64.55; | H, 4.12; | N, 6.74. |

EXAMPLE 6

Preparation of {9-[(3-trifluoromethylphenyl)methyl]-5-carbamoylcarbazol-4-yl}oxyacetic acid, sodium salt

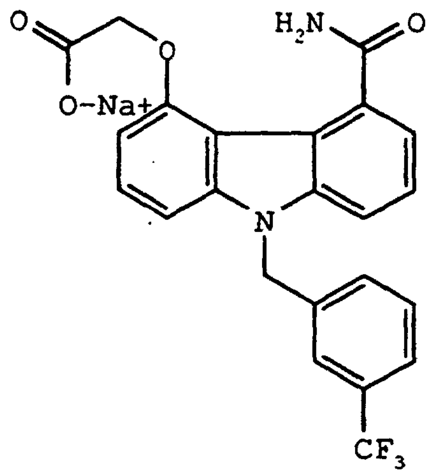

A. 9-[(3-Trifluoromethylphenyl)methyl]-5-carbomethoxy-1,2-dihydrocarbazol-4(3H)-one

40% Methanolic Triton B (2.18 mL, 4.8 mM) was slowly added dropwise to a solution of 5-carbomethoxy-1,2-dihydro-9H-carbazol-4(3H)-one (973 mg, 4.0 mM) in 10 mL of DMF at -10 °C. After 30 minutes, 3-(trifluoromethyl)benzyl chloride (1.53 g, 6.0 mM) and sodium iodide (900 mg, 6.0 mM) were added and the resultant mixture stirred at room temperature for 25 hours. The mixture was diluted with ethyl acetate, washed five times with $H_2O$, 1 N HCl, $H_2O$, sat $NaHCO_3$, and saturated brine, dried over anhydrous magnesium sulfate, filtered, concentrated, and dried *in vacuo*. The residue was purified by column chromatography on silica gel (elution with gradient methylene chloride/ethyl acetate) to afford 1.02 g (63%) of the 9-[(3-trifluoromethylphenyl)methyl]-5-carbomethoxy -1,2-dihydrocarbazol-4(3H)-one as a tan solid. $^1H$ NMR ($CDCl_3$) δ 7.6 (d, 1H, J=8 Hz), 7.45-7.2 (m, 5H), 7.0 (d, 1H, J=8 Hz), 5.4 (s, 2H), 4.05 (s, 3H), 2.85 (t, 2H, J=6 Hz), 2.6 (t, 2H, J=6 Hz), and 2.2 (m, 2H). IR (KBr, $cm^{-1}$) 1727 and 1652. MS (ES) m/e 400, 402.

| Elemental Analyses for $C_{22}H_{18}NO_3F_3$: | | | | |
|---|---|---|---|---|
| Calculated | C, 65.83; | H, 4.52; | N, 3.49; | F, 14.20. |
| Found | C, 65.63; | H, 4.58; | N, 3.39; | F, 14.14. |

B. 9-[(3-Trifluoromethylphenyl)methyl]-4-hydroxy-5-carbomethoxy carbazole

A solution of the 9-[(3-trifluoromethylphenyl)methyl]-5-carbomethoxy-1,2-dihydrocarbazol-4(3H)-one (1.21 g, 3.00 mM) and 2,3-dichloro-5, 6-dicyano-1, 4-benzoquinone (764 mg, 3.3 mM) in 25 mL of toluene was stirred between 80-90 °C for 7 hours. The mixture was purified directly by column chromatography on silica gel (elution with methylene chloride) to afford 340.0 mg (28%) of the 9-[(3-trifluoromethylphenyl)methyl]-4-hydroxy-5-carbomethyoxy carbazole as a yellow solid. $^1H$ NMR ($CDCl_3$) δ 10.35 (s, 1H), 8.0 (d, 1H, J=8 Hz), 7.6-7.3 (m, 6H), 7.05 (d, 1H, J=8 Hz), 6.85 (m, 2H), 5.6 (s, 2H), and 4.1 (s, 3H). IR ($CHCl_3$, $cm^{-1}$) 3378 and 1712. MS (ES) m/e 398, 400.

| Elemental Analyses for $C_{22}H_{16}NO_3F_3$: | | | |
|---|---|---|---|
| Calculated | C, 66.17; | H, 4.04; | N, 3.51. |
| Found | C, 66.99; | H, 4.12; | N, 3.53; F. |

C. 9-[(3-Trifluoromethylphenyl)methyl]-4-hydroxy-5-carbamoyl carbazole

A solution of the 9-[(3-trifluoromethylphenyl)methyl]-4-hydroxy-5-carbomethoxy carbazole (250 mg, 0.625

mM) in 5 mL THF and 20 mL concentrated aqueous ammonium hydroxide was sonicated for 30 h at 40-50 °C. The mixture was diluted with ethyl acetate and acidified to pH 1 with 5 N HCl. The aqueous layer was extracted three times with ethyl acetate. The combined organic extracts were washed with saturated brine, dried over magnesium sulfate, filtered, and concentrated. The residue was purified by column chromatography on silica gel (elution with gradient methylene chloride/ethyl acetate) to afford 120 mg (50%) of the 9-[(3-trifluoromethylphenyl)methyl]-4-hydroxy-5-carbamoyl carbazole as a white solid. $^1H$ NMR (DMSO-d6) δ 10.5 (s, 1H), 8.8 (br s, 1H), 8.4 (br s, 1H), 7.8 (d, 1H, J=8 Hz), 7.6-7.5 (m, 5H), 7.3 (t, 1H, J=8 Hz), 7.15 (d, 1H, J=8 Hz), 7.1 (d, 1H, J=8 Hz), 6.6 (d, 1H, J=8 Hz), and 5.8 (s, 2H). IR (KBr, $cm^{-1}$) 3429, 3206, and 1630. MS (ES) m/e 383, 385.

| Elemental Analyses for $C_{21}H_{15}N_2O_2F_3$: | | | |
|---|---|---|---|
| Calculated | C, 65.62; | H, 3.93; | N, 7.29. |
| Found | C, 67.50; | H, 4.00; | N, 7.19. |

D. {9-[(3-Trifluoromethylphenyl)methyl]-5-carbamoylcarbazol-4-yl)oxyacetic acid, methyl ester

40% Methanolic Triton B (0.18 mL, 0.4 mM) was added to a solution of the 9-[(3-trifluoromethylphenyl)methyl]-4-hydroxy-5-carbamoyl carbazole (115 mg, 0.3 mM) in 5 mL DMF at room temperature. After 15 minutes, methyl bromoacetate (95 mg, 0.6 mM) was added and the resultant mixture stirred at room temperature for 22 hours. The mixture was diluted with ethyl acetate, washed four times with $H_2O$, 1 N HCl, $H_2O$, sat. $NaHCO_3$, and saturated brine, dried over magnesium sulfate, filtered, and concentrated. The residue was purified by column chromatography on silica gel (elution with ethyl acetate) to afford 120 mg (88%) of the {9-[(3-trifluoromethylphenyl)methyl]-5-carbamoylcarbazol-4-yl)oxyacetic acid, methyl ester as a white solid. $^1H$ NMR ($CDCl_3$) δ 7.5-7.2 (m, 7H), 7.1 (d, 1H, J=8 Hz), 7.0 (d, 1H, J=8 Hz), 6.6 (d, 1H, J=8 Hz), 6.4 (br s, 1H), 6.0 (br s, 1H), 5.55 (s, 2H), 4.9 (s, 2H), and 3.9 (s, 3H). IR (KBr, $cm^{-1}$) 1763 and 1673. MS (ES) m/e 457.

| Elemental Analyses for $C_{24}H_{19}N_2O_4F_3$: | | | |
|---|---|---|---|
| Calculated | C, 63.16; | H, 4.20; | N, 6.14. |
| Found | C, 61.37; | H, 4.19; | N, 5.77. |

E. {9-[(3-Trifluoromethylphenyl)methyl]-5-carbamoylcarbazol-4-yl}oxyacetic acid, sodium salt.

A solution of the {9-[(3-trifluoromethylphenyl)methyl]-5-carbamoylcarbazol-4-yl}oxyacetic acid, methyl ester (91 mg, 0.153 mM) and 0.22 mL (0.22 mM) of 1 N NaOH in 8 mL of ethanol was stirred for 17 h at 25 °C. The ethanol was removed *in vacuo*. The resultant white precipitate was collected by filtration, washed with small amounts of EtOH and diethyl ether, then dried *in vacuo* to afford 75 mg (81%) of the {9-[(3-trifluoromethylphenyl)methyl]-5-carbamoylcarbazol-4-yl}oxyacetic acid, sodium salt as a white powder. $^1H$ NMR (DMSO-d6) δ 7.65 (s, 1H), 7.6 (m, 4H), 7.45 (t, 1H, J=8 Hz), 7.35 (t, 1H, J=8 Hz), 7.3 (t, 1H, J=8 Hz), 7.2 (d, 1H, J=8 Hz), 7.1 (d, 1H, J=8 Hz), 7.05 (d, 1H, J=8 Hz), 6.5 (d, 1H, J=8 Hz), 5.75 (s, 2H), and 4.3 (s, 2H). IR (KBr, $cm^{-1}$) 1665 and 1618. MS (ES) m/e 441, 443.

| Elemental Analyses for $C_{23}H_{16}N_2O_4F_3Na$: | | | |
|---|---|---|---|
| Calculated | C, 59.49; | H, 3.47; | N, 6.03. |
| Found | C, 60.69; | H, 3.78; | N, 5.75. |

EXAMPLE 7

Preparation of {9-[(2-methylphenyl)methyl}-5-carbamoylcarbazol-4-yl}oxyacetic acid, sodium salt

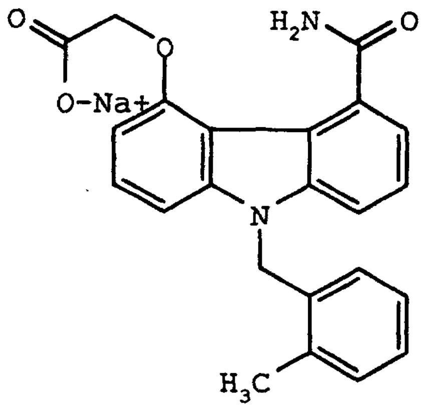

A. 9-[(2-Methylphenyl)methyl]-5-carbomethoxy-1,2-dihydrocarbazol-4(3H)-one

A suspension of 5-carbomethoxy-1,2-dihydro-9H-carbazol-4(3H)-one (870 mg, 3.58 mM), a-bromo-*o*-xylene (662 mg, 3.58 mM), and potassium carbonate (500 mg, 3.61 mM) in 20 mL DMF was stirred at room temperature for 20 hours. The mixture was diluted with ethyl acetate, washed with $H_2O$ and saturated brine, dried over anhydrous magnesium sulfate, filtered, concentrated to afford 1.21 g (98%) of the 9-[(2-methylphenyl)methyl]-5-carbomethoxy-1,2-dihydrocarbazol-4(3H)-one as a dark oil. $^1H$ NMR (DMSO-d6) δ 7.5-7.2 (m, 4H), 7.15 (t, 1H, J=8 Hz), 7.0 (t, 1H, J=8 Hz), 6.15 (d, 1H, J=8 Hz), 5.55 (s, 2H), 3.85 (s, 3H), 2.6 (m, 2H), 2.4 (m, 2H), 2.4 (s, 3H), and 2.1 (m, 2H). IR ($CHCl_3$, $cm^{-1}$) 3010, 2952, 1724, 1671, 1653, 1604, 1460, 1444, 1290, 1174, and 1122. MS (ES) m/e 348.5.

| Elemental Analyses for $C_{22}H_{21}NO_3$: | | | |
|---|---|---|---|
| Calculated: | C, 76.08; | H, 6.05; | N, 4.03. |
| Found | C, 73.33; | H, 6.36; | N, 4.30. |

B. 9-[(2-Methylphenyl)methyl]-4-hydroxy-5-carbomethoxy carbazole

A solution of the 9-[(2-methylphenyl)methyl]-5-carbomethoxy-1,2-dihydrocarbazol-4(3H)-one (1.2 g, 3.5 mM) and 2,3-dichloro-5,6-dicyano-1,4-benzoquinone (800 mg, 3.6 mM) in 70 mL of toluene was stirred at 80-90 °C for 5 hours. The mixture was purified directly by column chromatography on silica gel (elution with methylene chloride) to afford 260 mg (22%) of the 9-[(2-methylphenyl)methyl]-4-hydroxy-5-carbomethyoxy carbazole as a yellow solid. $^1H$ NMR (DMSO-d6) δ 10.25 (s, 1H), 7.5 (d, 1H, J=8 Hz), 7.4 (t, 1H, J=8 Hz), 7.3-7.1 (m, 4H), 6.9 (m, 2H), 6.6 (d, 1H, J=8 Hz), 6.1 (d, 1H, J=8 Hz), 5.65 (s, 2H), 3.8 (s, 3H), and 2.5 (s, 3H). IR (KBr, $cm^{-1}$) 3200, 1672, 1440, 1426, 1332, 1302, 1265, 1216, 1141, 761, 749, and 718. MS (ES) m/e 344, 346.

| Elemental Analyses for $C_{22}H_{19}NO_3$: | | | |
|---|---|---|---|
| Calculated | C, 76.52; | H, 5.51; | N, 4.06. |
| Found | C, 76.44; | H, 5.66; | N, 3.94. |

C. 9-[(2-Methylphenyl)methyl]-4-hydroxy-5-carbamoyl carbazole

A solution of the 9-[(2-methylphenyl)methyl]-4-hydroxy-5-carbomethoxy carbazole (260 mg, 0.75 mM) in 10 mL THF and 30 mL concentrated aqueous ammonium hydroxide was sonicated for 5 hours at 40-50 °C. The mixture was diluted with ethyl acetate and acidified to pH 1 with 5 N HCl. The aqueous layer was extracted three times with ethyl acetate. The combined organic extracts were washed with $H_2O$ and saturated brine, dried over magnesium sulfate, filtered, and concentrated. The residue was purified by column chromatography on silica gel (elution with gradient hexanes/ethyl acetate) to afford 90 mg (36%) of the 9-[(2-methylphenyl)methyl]-4-hydroxy-5-carbamoyl carbazole as a tan solid. $^1H$ NMR (DMSO-d6) δ 10.5 (s, 1H), 8.8 (br s, 1H), 8.4 (br s, 1H), 7.7 (m,

1H), 7.5 (m, 2H), 7.3 (m, 2H), 7.1 (t, 1H, J=8 Hz), 6.95 (d, 1H, J=8 Hz), 6.85 (t, 1H, J=8 Hz), 6.6 (d, 1H, J=8 Hz), 5.95 (d, 1H, J=8 Hz), 5.7 (s, 2H), and 2.5 (s, 3H). IR (KBr, $cm^{-1}$) 3451, 3191, 1627, 1600, 1584, 1562, 1435, 1329, 1322, 1263, and 774. MS (ES) m/e 329, 331.

| Elemental Analyses for $C_{21}H_{18}N_2O_2$: | | | |
|---|---|---|---|
| Calculated | C, 76.36; | H, 5.45; | N, 8.48. |
| Found | C, 75.66; | H, 5.79; | N, 8.07. |

D. {9-[(2-Methylphenyl)methyl]-5-carbamoylcarbazol-4-yl}oxyacetic acid, methyl ester

40% Methanolic Triton B (0.45 mL, 0.99 mM) was added to a solution of the 9-[(2-methylphenyl)methyl]-4-hydroxy-5-carbamoyl carbazole (80 mg, 0.24 mM) in 8 mL DMF at room temperature. After 3 minutes, methyl bromoacetate (115 mg, 0.72 mM) was added and the resultant mixture stirred at room temperature for 48 hours. The mixture was diluted with ethyl acetate, washed with $H_2O$, 1 N HCl, $H_2O$, and saturated brine, dried over magnesium sulfate, filtered, and concentrated. The residue was purified by column chromatography on silica gel (elution with ethyl acetate) to afford 80 mg (82%) of the {9-[(2-methylphenyl)methyl]-5-carbamoylcarbazol-4-yl}oxyacetic acid, methyl ester as a white solid. $^1H$ NMR (DMSO-d6) δ 7.56 (br s, 1H), 7.5-7.1 (m, 9H), 6.9 (t, 1H, J=8 Hz), 6.6 (d, 1H, J=8 Hz), 5.65 (s, 2H), 4.9 (s, 2H), 3.8 (s, 3H), and 2.5 (s, 3H). IR (KBr, $cm^{-1}$) 3367, 3153, 1760, 1740, 1672, 1644, 1619, 1591, 1578, 1498, 1456, 1425, 1327, 1200, 1153, 1109, 1100, and 777. MS (FD) m/e 402.

| Elemental Analyses for $C_{24}H_{22}N_2O_4$: | | | |
|---|---|---|---|
| Calculated | C, 71.64; | H, 5.47; | N, 6.96. |
| Found | C, 71.51; | H, 5.56; | N, 6.67. |

E. {9-[(2-Methylphenyl)methyl]-5-carbamoylcarbazol-4-yl}oxyacetic acid, sodium salt

A suspension of the {9-[(2-methylphenyl)methyl]-5-carbamoylcarbazol-4-yl}oxyacetic acid, methyl ester (15.5 mg, 0.039 mM) and 0.04 mL (0.04 mM) of 1 N NaOH in 5 mL of ethanol was stirred for 24 hours at 25 °C. The resultant white precipitate was collected by filtration, washed with a small amount of EtOH, then dried in *vacuo* to afford 10 mg (63%) of the {9-[(2-methylphenyl)methyl]-5-carbamoylcarbazol-4-yl}oxyacetic acid, sodium salt as a white powder. $^1H$ NMR (DMSO-d6) δ 7.55 (br s, 1H), 7.5-7.0 (m, 7H), 6.9 (d, 1H, J=8 Hz), 6.85 (t, 1H, J=8 Hz), 6.6 (d, 1H, J=8 Hz), 6.2 (d, 1H, J=8 Hz), 5.6 (s, 2H), 4.35 (s, 2H), and 2.5 (s, 3H). IR (KBr, $cm^{-1}$) 3390, 1656, 1613, 1595, 1573, 1498, 1455, 1408, 1325, 1332, and 719. MS (ES) m/e 387, 389.

| Elemental Analyses for $C_{23}H_{19}N_2O_4$: | | | |
|---|---|---|---|
| Calculated | C, 67.32; | H, 4.63; | N, 6.83. |
| Found | C, 64.72; | H, 4.44; | N, 6.40. |

EXAMPLE 8

Preparation of {9-[(3-methylphenyl)methyl]-5-carbamoylcarbazol-4-yl}oxyacetic acid, sodium salt

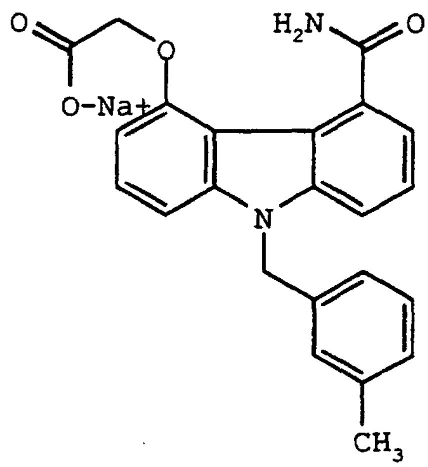

A. 9-[(3-Methylphenyl)methyl]-5-carbomethoxy-1,2-dihydrocarbazol-4(3H)-one

A suspension of 5-carbomethoxy-1,2-dihydro-9H-carbazol-4(3H)-one (870 mg, 3.58 mM), a-bromo-*m*-xylene (662 mg, 3.58 mM), and potassium carbonate (500 mg, 3.61 mM) in 20 mL DMF was stirred at room temperature for 16 hours. The mixture was diluted with ethyl acetate, washed with $H_2O$ and saturated brine, dried over anhydrous magnesium sulfate, filtered, concentrated to afford 1.18 g (95%) of the 9-[(3-methylphenyl)methyl]-5-carbomethoxy-1,2-dihydrocarbazol-4(3H)-one as a dark oil. $^1H$ NMR (DMSO-d6) δ 7.65 (dd, 1H, J=1 and 8 Hz), 7.3-7.1 (m, 3H), 7.05 (d, 1H, J=8 Hz), 7.0 (s, 1H), 6.85 (d, 1H, J=8 Hz), 5.5 (s, 2H), 3.8 (s, 3H), 3.0 (m, 2H), 2.45 (m, 2H), 2.3 (s, 3H), and 2.1 (m, 2H). IR ($CHCl_3$, $cm^{-1}$) 3010, 2953, 1724, 1652, 1605, 1465, 1442, 1288, 1174, and 1119. MS (ES) m/e 348.5.

| Elemental Analyses for $C_{22}H_{21}NO_3$: | | | |
|---|---|---|---|
| Calculated | C, 76.08; | H, 6.05; | N, 4.03. |
| Found | C, 74.53; | H, 6.03; | N, 3.68. |

B. 9-[(3-Methylphenyl)methyl]-4-hydroxy-5-carbomethoxy carbazole

A solution of the 9-[(3-methylphenyl)methyl]-5-carbomethoxy-1,2-dihydrocarbazol-4(3H)-one (1.18 g, 3.4 mM) and 2,3-dichloro-5,6-dicyano-1,4-benzoquinone (800 mg, 3.6 mM) in 70 mL of toluene was stirred at 80-90 °C for 6 hours. The mixture was purified directly by column chromatography on silica gel (elution with methylene chloride) to afford 300 mg (26%) of the 9-[(3-methylphenyl)methyl]-4-hydroxy-5-carbomethyoxy carbazole as a yellow solid. $^1H$ NMR (DMSO-d6) δ 10.2 (s, 1H), 7.65 (d, 1H, J=8 Hz), 7.35 (t, 1H, J=8 Hz), 7.25 (t, 1H, J=8 Hz), 7.2-7.0 (m, 4H), 6.9 (m, 2H), 6.6 (d, 1H, J=8 Hz), 5.6 (s, 2H), 3.85 (s, 3H), and 2.2 (s, 3H). IR (KBr, $cm^{-1}$) 3200, 1673, 1596, 1440, 1426, 1394, 1265, 1216, 1152, 750, 711, and 694. MS (ES) m/e 344, 346.

| Elemental Analyses for $C_{22}H_{19}NO_3$: | | | |
|---|---|---|---|
| Calculated | C, 76.52; | H, 5.51; | N, 4.06. |
| Found | C, 76.22; | H, 5.55; | N, 3.97. |

C. 9-[(3-Methylphenyl)methyl]-4-hydroxy-5-carbamoyl carbazole

A solution of the 9-[(3-methylphenyl)methyl]-4-hydroxy-5-carbomethoxy carbazole (300 mg, 0.87 mM) in 10 mL THF and 30 mL concentrated aqueous ammonium hydroxide was sonicated for 5 hours at 40-50 °C. The mixture was diluted with ethyl acetate and acidified to pH 1 with 5 N HCl. The aqueous layer was extracted three times with ethyl acetate. The combined organic extracts were washed with $H_2O$ and saturated brine, dried over

magnesium sulfate, filtered, and concentrated. The residue was purified by column chromatography on silica gel (elution with gradient hexanes/ethyl acetate) to afford 114 mg (40%) of the 9-[(3-methylphenyl)methyl]-4-hydroxy-5-carbamoyl carbazole as an off-white solid. $^1$H NMR (DMSO-d6) δ 10.5 (s, 1H), 8.8 (br s, 1H), 8.4 (br s, 1H), 7.8 (dd, 1H, J=1 and 8 Hz), 7.4 (m, 2H), 7.3 (t, 1H, J=8 Hz), 7.15-7.0 (m, 3H), 6.85 (d, 1H, J=8 Hz), 6.6 (d, 1H, J=8 Hz), 5.95 (d, 1H, J=8 Hz), 5.65 (s, 2H), and 2.25 (s, 3H). IR (KBr, cm$^{-1}$) 3434, 3203, 1629, 1599, 1579, 1552, 1443, 1330, 1262, 1214, and 776. MS (ES) m/e 329, 331.

| Elemental Analyses for $C_{21}H_{18}N_2O_2$: | | | |
|---|---|---|---|
| Calculated | C, 76.36; | H, 5.45; | N, 8.48. |
| Found | C, 77.56; | H, 5.67; | N, 8.26. |

D. {9-[(3-Methylphenyl)methyl]-5-carbamoylcarbazol-4-yl}oxyacetic acid, methyl ester

40% Methanolic Triton B (0.45 mL, 0.99 mM) was added to a solution of the 9-[(3-methylphenyl)methyl]-4-hydroxy-5-carbamoyl carbazole (100 mg, 0.30 mM) in 8 mL DMF at room temperature. After 3 minutes, methyl bromoacetate (115 mg, 0.72 mM) was added and the resultant mixture stirred at room temperature for 24 hours. The mixture was diluted with ethyl acetate, washed with $H_2O$, and saturated brine, dried over magnesium sulfate, filtered, and concentrated. The residue was purified by column chromatography on silica gel (elution with ethyl acetate) to afford 80 mg (66%) of the {9-[(3-methylphenyl)methyl]-5-carbamoylcarbazol-4-yl}oxyacetic acid, methyl ester as a white solid. $^1$H NMR (DMSO-d6) δ 7.6 (d, 1H, J=8 Hz), 7.55 (br s, 1H), 7.45-7.0 (m, 8H), 6.9 (d, 1H, J=8 Hz), 6.6 (d, 1H, J=8 Hz), 5.65 (s, 2H), 4.9 (s, 2H), 3.75 (s, 3H), and 2.2 (s, 3H). IR (KBr, cm$^{-1}$) 3367, 3157, 1760, 1642, 1589, 1499, 1455, 1424, 1328, 1216, 1151, 1102, 772, and 714. MS (FD) m/e 402.

| Elemental Analyses for $C_{24}H_{22}N_2O_4$: | | | |
|---|---|---|---|
| Calculated | C, 71.64; | H, 5.47; | N, 6.96. |
| Found | C, 71.01; | H, 5.60; | N, 6.66. |

E. {9-[(3-Methylphenyl)methyl]-5-carbamoylcarbazol-4-yl}oxyacetic acid, sodium salt

A suspension of the {9-[(3-methylphenyl)methyl]-5-carbamoylcarbazol-4-yl}oxyacetic acid, methyl ester (15.8 mg, 0.039 mM) and 0.04 mL (0.04 mM) of 1 N NaOH in 5 mL of ethanol was stirred for 24 h at 25 °C. The resultant white precipitate was collected by filtration, washed with a small amount of EtOH, then dried *in vacuo* to afford 10 mg (62%) of the {9-[(3-methylphenyl)methyl]-5-carbamoylcarbazol-4-yl}oxyacetic acid, sodium salt as a white powder. $^1$H NMR (DMSO-d6) δ 7.55 (d, 1H, J=8 Hz), 7.5-7.0 (m, 9H), 6.85 (d, 1H, J=8 Hz), 6.55 (d, 1H, J=8 Hz), 5.6 (s, 2H), 4.35 (s, 2H), and 2.2 (s, 3H). IR (KBr, cm$^{-1}$) 3390, 1656, 1613, 1595, 1573, 1498, 1455, 1408, 1325, 1332, and 719. MS (ES) m/e 387, 389.

| Elemental Analyses for $C_{23}H_{19}N_2O_4Na$: | | | |
|---|---|---|---|
| Calculated | C, 67.32; | H, 4.63; | N, 6.83. |
| Found | C, 61.20; | H, 4.64; | N, 6.06. |

EXAMPLE 9

Preparation of {9-[(3-trifluoromethoxyphenyl)methyl]-5-carbamoylcarbazol-4-yl}oxyacetic acid, sodium salt

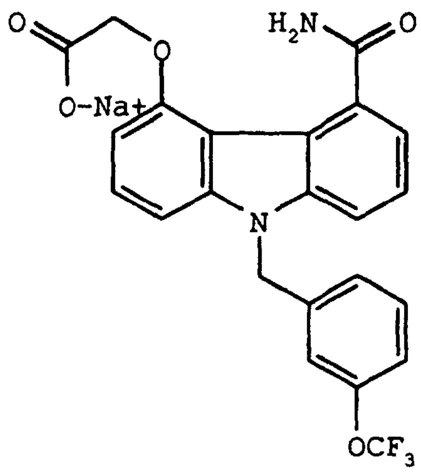

A. 9-[(3-Trifluoromethoxyphenyl)methyl]-5-carbomethoxy-1,2-dihydrocarbazol-4(3H)-one

A suspension of 5-carbomethoxy-1,2-dihydro-9H-carbazol-4(3H)-one (935 mg, 3.85 mM), 3-trifluoromethoxybenzyl bromide (1.0 g, 3.93 mM), and potassium carbonate (531 mg, 3.85 mM) in 20 mL DMF was stirred at room temperature for 17 hours. The mixture was diluted with ethyl acetate, washed with $H_2O$ and saturated brine, dried over anhydrous magnesium sulfate, filtered, concentrated to afford 1.6 g (100%) of the 9-[(3-trifluoromethoxyphenyl)methyl]-5-carbomethoxy-1,2-dihydrocarbazol-4(3H)-one as a foam. $^1H$ NMR (DMSO-d6) δ 7.7 (dd, 1H, J=1 and 8 Hz), 7.45 (t, 1H, J=8 Hz), 7.3-7.1 (m, 4H), 7.05 (d, 1H, J=8 Hz), 5.6 (s, 2H), 3.8 (s, 3H), 3.0 (m, 2H), 2.45 (m, 2H), and 2.1 (m, 2H). IR ($CHCl_3$, $cm^{-1}$) 1729, 1647, 1439, 1259, 1176, and 1116. MS (ES) m/e 418.

| Elemental Analyses for $C_{22}H_{18}NO_4F_3$: | | | |
|---|---|---|---|
| Calculated | C, 63.31; | H, 4.32; | N, 3.36. |
| Found | C, 63.12; | H, 4.35; | N, 3.31. |

B. 9-[(3-Trifluoromethoxyphenyl)methyl]-4-hydroxy-5-carbomethoxy carbazole

A solution of the 9-[(3-trifluoromethoxyphenyl)methyl]-5-carbomethoxy-1,2-dihydrocarbazol-4(3H)-one (0.75 g, 1.8 mM) and 2,3-dichloro-5,6-dicyano-1,4-benzoquinone (490 mg, 2.16 mM) in 70 mL of toluene was stirred at reflux for 6 hours. The mixture was purified directly by column chromatography on silica gel (elution with methylene chloride) to afford 300 mg (40%) of the 9-[(3-trifluoromethoxyphenyl)methyl]-4-hydroxy-5-carbomethyoxy carbazole as a yellow solid. $^1H$ NMR (DMSO-d6) δ 10.25 (s, 1H), 7.7 (d, 1H, J=8 Hz), 7.5-7.0 (m, 8H), 6.6 (d, 1H, J=8 Hz), 5.7 (s, 2H), and 3.85 (s, 3H). IR (KBr, $cm^{-1}$) 3200, 1673, 1441, 1268, 1217, 1173, and 753. MS (ES) m/e 414, 416.

| Elemental Analyses for $C_{22}H_{16}NO_3F_3$ | | | |
|---|---|---|---|
| Calculated | C, 63.61; | H, 3.86; | N, 3.37. |
| Found | C, 63.40; | H, 3.99; | N, 3.43. |

C. 9-[(3-Trifluoromethoxyphenyl)methyl]-4-hydroxy-5-carbamoyl carbazole

A solution of the 9-[(3-trifluoromethoxyphenyl)methyl]-4-hydroxy-5-carbomethoxy carbazole (260 mg, 0.62 mM) in 10 mL THF and 30 mL concentrated aqueous ammonium hydroxide was stirred vigorously for 132 hours. The mixture was diluted with ethyl acetate and acidified to pH 1 with 5 N HCl. The aqueous layer was extracted three times with ethyl acetate. The combined organic extracts were washed with $H_2O$ and saturated brine, dried over magnesium sulfate, filtered, and concentrated. The residue was purified by column chromatography on silica

gel (elution with gradient hexanes/ethyl acetate) to afford 150 mg (60%) of the 9-[(3-trifluoromethoxyphenyl)methyl]-4-hydroxy-5-carbamoyl carbazole as an off-white solid. $^1$H NMR (DMSO-d6) δ 10.5 (s, 1H), 8.8 (br s, 1H), 8.4 (br s, 1H), 7.85 (dd, 1H, J=1 and 8 Hz), 7.5-7.15 (m, 5H), 7.1 (d, 1H, J=8 Hz), 7.0 (d, 1H, J=8 Hz), 6.6 (d, 1H, J=8 Hz), 5.95 (d, 1H, J=8 Hz), and 5.65 (s, 2H). IR (KBr, $cm^{-1}$) 3431, 3203, 1629, 1601, 1580, 1548, 1446, 1330, 1261, 1215, and 777. MS (ES) m/e 399, 401.

| Elemental Analyses for $C_{21}H_{15}N_2O_2F_3$: | | | |
|---|---|---|---|
| Calculated | C, 63.00; | H, 3.75; | N, 7.0. |
| Found | C, 63.15; | H, 4.07; | N, 6.84. |

D. {9-[(3-Trifluoromethoxyphenyl)methyl]-5-carbamoylcarbazol-4-yl}oxyacetic acid, methyl ester

40% Methanolic Triton B (0.15 mL, 0.34 mM) was added to a solution of the 9-[(3-trifluoromethoxyphenyl) methyl]-4-hydroxy-5-carbamoyl carbazole (115 mg, 0.28 mM) in 8 mL DMF at room temperature. After 3 minutes, methyl bromoacetate (65 mg, 0.41 mM) was added and the resultant mixture stirred at room temperature for 23 hours. The mixture was diluted with ethyl acetate, washed with $H_2O$, and saturated brine, dried over magnesium sulfate, filtered, and concentrated. The residue was purified by column chromatography on silica gel (elution with ethyl acetate) to afford 112 mg (83%) of the {9-[(3-trifluoromethoxyphenyl)methyl]-5-carbamoylcarbazol-4-yl}oxyacetic acid, methyl ester as a white solid. $^1$H NMR (DMSO-d6) δ 7.6 (d, 1H, J=8 Hz), 7.55 (br s, 1H), 7.5-7.0 (m, 9H), 6.6 (d, 1H, J=8 Hz), 5.7 (s, 2H), 4.9 (s, 2H), and 3.75 (s, 3H). IR (KBr, $cm^{-1}$) 3488, 3141, 1763, 1674, 1501, 1444, 1269, 1215, 1178, 1102, 772, and 714. MS (FD) m/e 472.

| Elemental Analyses for $C_{24}H_{19}N_2O_5F_3$: | | | |
|---|---|---|---|
| Calculated | C, 61.02; | H, 4.03; | N, 5.93. |
| Found | C, 61.05; | H, 4.17; | N, 5.81. |

E. {9-[(3-Trifluoromethoxyphenyl)methyl]-5-carbamoylcarbazol-4-yl}oxyacetic acid, sodium salt

A suspension of the {9-[(3-trifluoromethoxyphenyl)methyl]-5-carbamoylcarbazol-4-yl}oxyacetic acid, methyl ester (22.4 mg, 0.047 mM) and 0.065 mL (0.065 mM) of 1 N NaOH in 5 mL of ethanol was stirred for 24 hours at 25 °C. The solvent was removed *in vacuo* and the residue suspended in EtOH. The resultant white precipitate was collected by filtration, washed with a small amount of EtOH, then dried *in vacuo* to afford 9 mg (41%) of the {9-[(3-trifluoromethoxyphenyl)methyl]-5-carbamoylcarbazol-4-yl}oxyacetic acid, sodium salt as a white powder. MS (ES) m/e 457, 459.

Example 10

Preparation of [9-benzyl-5-carbamoyl-1-methylcarbazol-4-yl]oxyacetic acid

A. Preparation of 5-carbamoyl-4-methoxy-1-methylcarbazole

A solution of 0.805 g of 9-benzyl-5-methoxy-8-methyl-1,2,3,4-tetrahydrocarbazole-4-carboxamide in 24 ml of carbitol was treated with 1.1 g of 5% palladium on carbon and was refluxed for 6 hours open to the air. After cooling, the solution was filtered thourough a pad of celite and the pad was washed with ethyl acetate. The filtrates were diluted with ether and washed four times with water and dried over magnesium sulfate and concentrated. The residue was chromatographed on silica gel using methanol/0-4% in dichloromethane to afford 0.166 g (28%) of debenzylated carbazole. ESIMS m/e 255 ($M^+$+1), 253 ($M^+$-1) NMR (300 MHz, $CDCl_3$): δ 8.13 (br, 1H); 7.51 (d, J=8.1, 1H); 7.40 (t, J=7.6, 1H); 7.32 (d, J=7.2, 1H); 7.18 (d, J=7.8, 1H); 6.60 (d, J=8.0, 1H); 5.68 (br, 2H); 3.99 (s, 3H); 2.50 (s 3H).

B. Preparation of 9-benzyl-5-carbamoyl-4-methoxy-1-methylcarbazole

A solution of 0.148 g of 5-carbamoyl-4-methoxy-1-methylcarbazole in 1.1 ml of dimethylformamide was added to 0.026 g of sodium hydride (60% in mineral oil) in 0.4 ml of dimethylformamide and stirred for 60 minutes at room temperature. Benzyl bromide (0.076 ml) was then added and the reaction was stirred overnight. The reaction mixture was poured into 20 ml of saturated ammonium chloride solution and then extracted twice with ethyl acetate. The extracts were washed with water and then with brine, dried over magnesium sulfate and concentrated. The residue was rinsed with hexane and dissolved in dichloromethane, filtered and concentrated to afford 0.21 g of

the subtitle compound. FDMS *m/e* 344 ($M^+$)

| Elemental Analyses | | | |
|---|---|---|---|
| Calculated | C 76.72; | H 5.85; | N 8.13 |
| Found | C 75.20; | H 6.19; | N 7.54 |

C. Preparation of [9-benzyl-5-carbamoyl-1-methylcarbazol-4-yl]oxyacetic acid methyl ester ]

A solution of 0.23 g of 9-benzyl-5-carbamoyl-4-methoxy-1-methylcarbazole in 4 ml of dimethylformamide was added to a 1 ml solution of sodium ethane thiolate (prepared from 0.116 g of sodium hydride 60% dispersion and 0.22 ml of ethanethiol under nitrogen) and heated at 110°C for 15 hours. The reaction mixture was cooled, poured into 20 ml of 1 N HCl and extracted twice with ethyl acetate. The extracts were washed twice with water and then with brine, dried over magnesium sulfate and concentrated. The residue was chromatographed on silica gel using methanol/0-1% in dichloromethane to afford 0.146 g (66%) of the demethylated intermediate. A solution of 0.146 g of this intermediate in 1.5 ml of dimethylformamide was added to 0.021 g of sodium hydride (60% in mineral oil) in 0.5 ml of dimethylformamide. After stirring for 10 minutes at room temperature, 0.054 ml of methyl bromoacetate was added. After stirring for 5 hours at room temperature, the reaction mixture was poured into water and extracted twice with ethyl acetate. The extracts were washed with water and then with brine, dried over magnesium sulfate and concentrated. The residue was chromatographed on silica gel using methanol/ 0-2% in dichloromethane to afford 0.10 g (56%) of the subtitle compound. mp. 228-230°C ESIMS m/e 403 ($M^+$+1)

| Elemental Analyses | | | |
|---|---|---|---|
| Calculated | C 71.63; | H 5.51; | N 6.96 |
| Found | C 71.34; | H 5.60; | N 6.70 |

D. Preparation of [9-benzyl-5-carbamoyl-l-methylcarbazol-4-yl]oxyacetic acid

A slurry of 32 mg (0.0795 mmol) of [9-benzyl-5-carbamoyl-1-methylcarbazol-4-yl]oxyacetic acid methyl ester in 1 ml of tetrahydrofuran and 3.5 ml of methanol was treated with 0.3 ml of an aqueous 2 N sodium hydroxide solution and stirred overnight at room temperature. The solvent was evaporated and the residue was partitioned between 1:1 ethyl acetate/tetrahydrofuran and 0.2 N HCl solution. After another extraction with 1:1 ethyl acetate/ tetrahydrofuran, the extracts were washed with brine, dried over magnesium sulfate and concentrated to afford (27 mg) of the title compound. mp. 253-254°C. ESIMS *m/e* 389 ($M^+$+1), 387 ($M^+$-1) NMR (300 MHz, $d^6$-DMSO): δ 12.83 (br, 1H); 7.75 (br, 1H); 7.53 (d, J=8.2, 1H); 7.41-7.34 (m, 2H); 7.28-7.17 (m, 3H); 7.07 (m, 2H); 6.90 (d, J=7.2, 2H); 6.49 (d, J=8,1, 1H); 5.89 (s, 2H); 4.79 (s, 2H); 2.52 (s, 3H).

Example 11

Preparation of [9-benzyl-4-carbamoyl-8-fluoro-1,2,3,4-tetrahydrocarbazol-5-yl]oxyacetic acid

A. Preparation of (2-chloro-4-fluorophenyl)- ethyl carbonate

A solution of 19.16 g of 2-chloro-4-fluorophenol in 65.4 ml of 2 N aqueous sodium hydroxide solution was cooled in an ice bath and treated dropwise with 16.3 ml of ethyl chloroformate. After stirring at room temperature overnight, the two-phase reaction mixture was diluted with 100 ml of water and extracted with 300 ml of a 1:1 pentane/ether mixture. The extract was washed three times with 0.02 N sodium hydroxide solution, water and then brine. After drying and evaporation, 27.63 g (97%) of the subtitle compound were obtained. NMR (300 MHz, $CDCl_3$): δ 7.23-7.18 (m, 2H); 7.00 (dt, J=8.4, 2.7, 1H); 4.35 (q, J=7.1, 2H); 1.40 (t, J=7.1, 3H).

B. Preparation of (2-chloro-4-fluoro-5-nitrophenyl)- ethyl carbonate

A solution of 27.63 g of (2-chloro-4-fluorophenyl)-ethyl carbonate in 60 ml of dichloromethane was cooled in an ice bath and treated dropwise with 31.86 g of a 1:2 mixture of fuming nitric acid (90%) and concentrated sulfuric acid. The reaction was stirred for 2 hours at room temperature and then cooled with ice and treated with another 4.5 g of the same nitrating mixture. The reaction was stirred overnight at room temperature, poured into 200 ml of ice and water,and extracted twice with dichloromethane. The extracts were washed with water and then with brine, dried over magnesium sulfate and concentrated to afford 33.01 g (99%) of the subtitle compound. mp. 50-51 C

| Elemental Analyses | | | | |
|---|---|---|---|---|
| Calculated | C 41.01; | H 2.68; | N 5.31; | Cl 13.45 |
| Found | C 41.03; | H 2.59; | N 5.38; | Cl 13.71 |

C. Preparation of 2-chloro-4-fluoro-5-nitroanisole

A solution of 15.0 g of (2-chloro-4-fluoro-5-nitrophenyl)- ethyl carbonate in 100 ml of dimethyl formamide was treated with 18.6 g of cesium carbonate, 7.1 ml of iodomethane and 7 ml of methanol and stirred overnight at room temperature. The reaction mixture was poured into water and extracted twice with ether. The extracts were washed twice with water and then with brine, dried over magnesium sulfate and concentrated to afford 11.4g of the subtitle compound. mp. 69-70°C Ex. 57, C.

| Elemental Analyses | | | | |
|---|---|---|---|---|
| Calculated | C 40.90; | H 2.45; | N 6.81; | Cl 17.25 |
| Found | C 41.20; | H 2.48; | N 6.70; | Cl 17.44 |

D. Preparation of 2-fluoro-5-methoxyaniline

A solution of 5.63 g of 2-chloro-4-fluoro-5-nitroanisole in 90 ml of ethanol and 5 ml of triethylamine was hydrogenated at room temperature under 60 pounds per square inch with 1.0 g of 5% palladium on carbon for four hours. The catalyst was filtered off and the solvent was evaporated. The residue was slurried in chloroform and filtered thourough a plug of silica gel and then evaporated. This residue was chromatographed on silica gel using hexane/chloroform mixtures to afford 2.77 g (72%) of the subtitle compound. mp. 253-254°C. NMR (300 MHz, $CDCl_3$): $\delta$ 6.88 (dd, J=10.6, 8.9, 1H); 6.32 (dd, J=7.4, 3.0, 1H); 6.20 (dt, J=8.9, 3.2,1H); 3.73 (s, 3H); 3.72 (br, 2H).

E. Preparation of N-benzyl-2-fluoro-5-methoxyaniline

This procedure was patterned after that of Tietze and Grote, Chem Ber. 126(12), 2733 (1993). A solution of 2.73 g of 2-fluoro-5-methoxyaniline and 2.67 g of benzaldehyde in 48 ml of methanol was treated with 3.43 g of zinc chloride and then cooled in an ice bath. Sodium cyanoborohydride (1.58 g) was added in small poroom temperature ions over 30 minutes and the reaction was stirred for five hours at room temperature. After evaporation of the solvent, the residue was slurried in 40 ml of 1 N sodium hydroxide solution and then extracted twice with ether. The extracts were washed with water and then with brine, dried over magnesium sulfate and concentrated. The residue was recrystallized from hexane to afford 2.61 g and the mother liquors were chromatographed on silica gel using 20:1 hexane/ether to afford another 1.4 g of the subtitle compound (90%). mp. 56-58°C

| Elemental Analyses | | | |
|---|---|---|---|
| Calculated | C 72.71; | H 6.10; | N 6.06 |
| Found | C 72.51; | H 6.06; | N 5.99 |

F. Preparation of ethyl 9-benzyl-5-methoxy-8-fluoro-1,2,3,4-tetrahydrocarbazole-4-carboxylate

A solution of 0.62 g of N-benzyl-2-fluoro-5-methoxyaniline in 20 ml of dry tetrahydrofuran was cooled in an ice bath and treated with 11.3 ml of 0.5 M potassium bis(trimethylsilyl)amide in toluene. After stirring for 30 minutes, 0.74 g of 2-carboethoxy-6-bromocyclohexanone (Sheehan and Mumaw, JACS, 72, 2127 (1950)) in 4 ml of tetrahydrofuran was added and the reaction was allowed to warm slowly to room temperature over two hours. The reaction was quenched with saturated ammonium chloride solution and extracted twice with ether. The extracts were washed with water and then with brine, dried over magnesium sulfate and concentrated. This residue was chromatographed on silica gel using hexane/ ether mixtures to afford 0.796 g (74%) of N-alkylated intermediate diastereomers. This mixture was refluxed in 20 ml of benzene with 0.99 g of zinc chloride overnight. The solvent was evaporated and the residue was partitioned between 25 ml of 1 N HCl and 25 ml of ethyl acetate and then extracted once more with ethyl acetate. The organic layers were washed with water and then brine, dried over magnesium sulfate and concentrated to afford 0.734 g (96%) of the subtitled compound. ESIMS m/e 382 ($M^+$+1)

| Elemental Analyses | | | |
|---|---|---|---|
| Calculated | C 72.42; | H 6.34; | N 3.67 |
| Found | C 72.20; | H 6.26; | N 3.70 |

G. Preparation of 9-benzyl-5-methoxy-8-fluoro-1,2,3,4-tetrahydrocarbazole-4-carboxamide

Ethyl 9-benzyl-5-methoxy-8-fluoro-1,2,3,4-tetrahydrocarbazole-4-carboxylate (0.722 g) was treated similarly as described in Example 49, Part C and chromatographed on silica gel using 1% methanol in dichloromethane to afford 0.482 g (72%) of the subtitle compound. ESIMS *m/e* 353 ($M^+$+1)

| Elemental Analyses | | | |
|---|---|---|---|
| Calculated | C 71.57; | H 6.01; | N 7.95 |
| Found | C 71.42; | H 5.83; | N 7.75 |

H. Preparation of [9-benzyl-4-carbamoyl-8-fluoro-1,2,3,4-tetrahydrocarbazol-5-yl]oxyacetic acid methyl ester

9-Benzyl-5-methoxy-8-fluoro-1,2,3,4-tetrahydrocarbazole-4-carboxamide (0.170 g) was converted similarly as described in Example 49, Part D and chromatographed on silica gel using methanol/ 0-1% in dichloromethane to afford 85 mg (50%) of the subtitle compound. mp. 183-185°C

| Elemental Analyses | | | |
|---|---|---|---|
| Calculated | C 67.31; | H 5.65; | N 6.82 |
| Found | C 67.58; | H 5.48; | N 6.95 |

I. Preparation of [9-benzyl-4-carbamoyl-8-fluoro-1,2,3,4-tetrahydrocarbazol-5-yl]oxyacetic acid

[9-Benzyl-4-carbamoyl-8-fluoro-1,2,3,4-tetrahydrocarbazol-5-yl]oxyacetic acid methyl ester (71 mg) was hydrolyzed similarly as described in Example 50, Part D to afford 65 mg of the title compound. ESIMS *m/e* 397 ($M^+$+1), 395 ($M^+$-1) NMR (300 MHz, $d^6$-DMSO): δ 13.03 (br, 1H); 7.31-7.19 (m, 3H); 6.97 (d, J=7.4, 2H); 6.95 (br, 1H); 6.70 (d, J=3.8, 1H); 6.67 (dd, J=12.4, 3.9, 1H); 6.28 (dd, J=8.5, 2.6, 1H); 5.39 (ABq, 2H); 4.64 (s, 2H); 3.92 (br, 1H); 2.71 (m, 1H); 2.44 (m, 1H); 2.02 (m, 2H); 1.76 (m, 2H).

Example 12

Preparation of [9-benzyl-5-carbamoyl-1-chlorocarbazol-4-yl]oxyacetic acid

A. Preparation of 9-benzyl-5-carbamoyl-4-methoxy-1-chlorocarbazole

A solution of 1.0 g of 9-benzyl-5-methoxy-8-methyl-1,2,3,4-tetrahydrocarbazole-4-carboxamide was oxidized similarly to Example 51, Part A and chromatographed on silica gel using dichloromethane/ 0-1% methanol to afford 0.66 g (67%) of the subtitle compound. FDMS m/e 364 ($M^+$)

| Elemental Analyses | | | | |
|---|---|---|---|---|
| Calculated | C 69.14; | H 4.70; | N 7.68; | Cl 9.72 |
| Found | C 69.40; | H 4.64; | N 7.49; | Cl 9.98 |

B. Preparation of 5-carbamoyl-4-hydroxy-l-chlorocarbazole

A solution of 0.66 g of 9-benzyl-5-carbamoyl-4-methoxy-1-chlorocarbazole in 40 ml of dichloromethane was cooled in an ice bath treated dropwise with 14 ml of 1.0 M boron tribromide solution in dichloromethane. The reaction was allowed to warm to room temperature slowly over 2 hours and then quenched by pouring into ice and then adding 50 ml of 1 N HCl. The mixture was extracted with dichloromethane (3x200 ml) and the extracts were washed with brine, dried with magnesium sulfate and concentrated. The aqueous layers exhibited a precipitate and was then extracted twice with ethyl acetate, washed with brine, dried with magnesium sulfate and concentrated to afford 0.287 g of the subtitle compound. The first residue was chromatographed on silica gel using 0.5% methanol in dichloromethane to afford another 93 mg of the subtitle compound. (total yield 80%) ESIMS *m/e* 259 ($M^+$-1) NMR (300 MHz, $d^6$-DMSO): δ 11.79 (s, 1H); 10.76 (s, 1H); 8.87 (br s, 1H); 8.41 (br s, 1H); 7.77 (t, J=4.6, 1H); 7.48 (d, J=4.2, 2H); 7.34 (d, J=8.5, 1H); 6.54 (d, J=8.5, 1H).

C. Preparation of [5-carbamoyl-l-chlorocarbazol-4-yl]oxyacetic acid methyl ester

A solution of 0.28 g of 5-carbamoyl-4-hydroxy-1-chlorocarbazole in 6 ml of tetrahydrofuran was added to 0.043 g of sodium hydride (60% in mineral oil) in 1 ml of tetrahydrofuran and stirred for 60 minutes at room temperature.

Methyl bromoacetate (0.11 ml) was then added and the reaction was stirred overnight. The reaction mixture was poured into 20 ml of saturated ammonium chloride solution and then extracted twice with ethyl acetate. The extracts were washed with water and then with brine, dried over magnesium sulfate and concentrated. The residue was chromatographed on silica gel eluting with chloroform and then 2:1 chloroform/ethyl acetate to afford 0.16 g (45%) of the subtitle compound. ESIMS m/e 333 ($M^{+}$+1), 335 ($M^{+}$+3), 331 ($M^{+}$-1) NMR (300 MHz, $d^{6-}$DMSO): δ 11.73 (s, 1H); 7.56 (d, J=8.1, 1H); 7.50 (br s, 1H); 7.43-7.35 (m, 2H); 7.18 (br s, 1H); 7.06 (d, J=7.8, 1H); 6.56 (d, J=8.6, 1H); 4.90 (s, 2H); 3.70 (s, 3H).

D. Preparation of [9-benzyl-5-carbamoyl-l-chlorocarbazol-4-yl]oxyacetic acid methyl ester

A solution of 78 mg of [5-carbamoyl-l-chlorocarbazol-4-yl]oxyacetic acid methyl ester in 0.8 ml of dry dimethylformamide was added to 10 mg sodium hydride (60% in mineral oil) in 0.2 ml of dimethylformamide and stirred for 15 minutes. Benzyl bromide (0.031 ml) was then added and the reaction was stirred overnight. The reaction mixture was poured into water and acidified with 1 ml of 1 N HCl solution and extracted twice with ethyl acetate. The extracts were washed with water (3x) and then with brine, dried over magnesium sulfate and concentrated. The residue was chromatographed on silica gel eluting with methanol/0-2% in dichloromethane to afford 40 mg of the subtitle compound. ESIMS *m/e* 423 ($M^{+}$+1) 425 ($M^{+}$+3) NMR (300 MHz, $CDCl_3$): δ 7.43-7.22 (m, 7H); 7.06 (d, J=7.3, 2H); 6.51 (d, J=8.6, 1H); 6.05 (s, 2H); 5.80 (br, 2H); 4.88 (s, 2H); 3.83 (s, 3H).

E. Preparation of [9-benzyl-5-carbamoyl-1-chlorocarbazol-4-yl]oxyacetic acid

[9-Benzyl-5-carbamoyl-1-chlorocarbazol-4-yl]oxyacetic acid methyl ester (15 mg) was hydrolyzed similarly as described in Example 50, Part D to afford 14 mg of the title compound. mp. 240-2°C ESIMS *m/e* 409 ($M^{+}$+1), 411 ($M^{+}$+3), 407 ($M^{+}$-1) NMR (300 MHz, $d^{6}$-DMSO): δ 12.94 (br, 1H); 7.70 (br, 1H); 7.61 (d, J=8.3, 1H); 7.43 (t, J=7.8, 1H); 7.36 (m, 2H); 7.28-7.19 (m, 3H); 7.13 (d, J=7.2, 1H); 6.99 (d, J=7.4, 2H); 6.63 (d, J=8.6, 1H); 6.08 (s, 2H); 4.83 (s, 2H).

EXAMPLE 13

Preparation of [9-[(Cyclohexyl)methyl]-5-carbamoylcarbazol-4-yl]oxyacetic acid

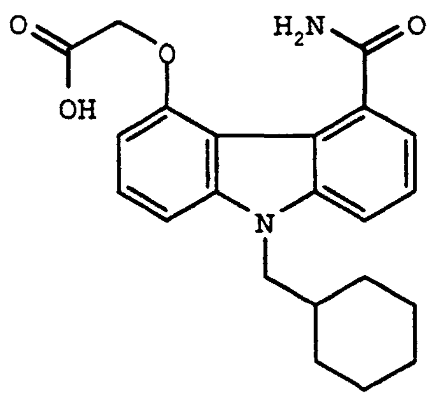

A. 9-[(Cyclohexyl)methyl]-5-carbomethoxy-1,2-dihydrocarbazol-4 (3H)-one

A 0 °C suspension of 5-carbomethoxy-1,2-dihydro-9H-carbazol-4(3H)-one (1.0 g, 4.11 mmol), a catalytic amount of NaI (ca. 10 mg) and $K_2CO_3$ (1.1 g, 8.22 mmol) in 10 mL of DMF was treated with cyclohexylmethyl bromide (0.631 mL, 4.52 mmol). After stirring overnight at ambient temperature, an additional 0.63 mL cyclohexylmethylbromide was added, and the resulting mixture was heated at 60 °C for 3 hours. The mixture was poured into $H_2O$ (30 mL) and extracted with EtOAc (2 x 25 mL). The combined organic layers were washed with $H_2O$ (4 x 50 mL), dried over anhydrous $Na_2SO_4$, filtered and concentrated *in vacuo*. The residue was purified by radial chromatography on silica gel (elution with a gradient of 20% to 40% EtOAc/hexanes) to afford 1.36 g (4.01 mmol; 97%) of 9-[(cyclohexyl)methyl]-5-carbomethoxy-1,2-dihydrocarbazol-4(3H)-one as a white foam. IR ($CHCl_3$, $cm^{-1}$) 3011, 2932, 2857, 1725, 1649, 1469, 1446, 1288 and 1120. MS (ES) m/e 340 (M+1), 453 ($M+AcO^{-}$). FAB HRMS m/e, Calcd for $C_{21}H_{26}NO_3$: 340.1913. Found: 340.1916 (M+1).

| Elemental Analyses for $C_{21}H_{25}NO_3$: | | | |
|---|---|---|---|
| Calculated | C, 74.31; | H, 7.42; | N, 4.13. |
| Found | C, 72.65; | H, 7.39; | N, 4.70. |

B. 9-[(Cyclohexyl)methyl]-4-hydroxy-5-carbomethoxy carbazole

A solution of 9-[(cyclohexyl)methyl]-5-carbomethoxy-1,2-dihydrocarbazol-4(3H)-one (1.16 g, 3.42 mmol) and 2,3-dichloro-5,6-dicyano-1,4-benzoquinone (853 mg, 3.76 mmol) in 20 mL of toluene was heated at 80 °C for 3 hours. The mixture was purified directly by column chromatography on silica gel (elution with $CH_2Cl_2$) to afford 259 mg (0.768 mmol; 22%) of 9-[(cyclohexyl)methyl]-4-hydroxy-5-carbomethoxy carbazole as a yellow oil which slowly solidified. MS (ES) m/e 338 (M+1), 336 (M-1). Elemental Analyses for $C_{21}H_{23}NO_3$:

| Elemental Analyses for $C_{21}H_{23}NO_3$: | | | |
|---|---|---|---|
| Calculated | C, 74.75; | H, 6.87; | N, 4.15. |
| Found | C, 74.95; | H, 6.99; | N, 4.42. |

C. 9-[(Cyclohexyl)methyl]-4-hydroxy-5-carbamoyl carbazole

A solution of 9-[(cyclohexyl)methyl]-4-hydroxy-5-carbomethoxy carbazole (205 mg, 0.608 mmol) in 5 mL of THF and 20 mL of concentrated aqueous ammonium hydroxide was treated with a stream of $NH_3$ gas to ensure saturation. The reaction vessel was capped, and the mixture was heated at 35 °C with stirring until tlc indicated complete consumption of starting material (20 hrs). The THF was evaporated, and the aqueous layer was filtered. The green solid precipitate was dissolved in THF and purified by radial chromatography on silica gel (elution with $CH_2Cl_2$). The resultant foam was triturated with ether to afford 138 mg (70%) of the title compound as an off-white solid. IR (KBr, $cm^{-1}$) 3418, 3200, 3131, 1629, 1600, 1443, 1261, 778. 'FAB HRMS m/e, Calcd for $C_{20}H_{23}N_2O_2$: 323.1760. Found: 323.1760 (M+1).

D. [9-[(Cyclohexyl)methyl]-5-carbamoylcarbazol-4-yl]oxyacetic acid, methyl ester

A mixture of 9-[(cyclohexyl)methyl]-4-hydroxy-5-carbamoyl carbazole (60 mg, 0.186 mmol) and $Cs_2CO_3$ (150 mg; 0.460 mmol) in 2 mL of DMF was treated with methyl bromoacetate (0.023 mL; 0.242 mmol). The reaction was stirred for 2 hours at ambient temperature, then it was diluted with EtOAc and $H_2O$ (10 mL each). The aqueous layer was saturated with solid NaCl and extracted with EtOAc (2 x 10 mL). The combined organic layers were washed with $H_2O$ (2 x 25 mL), dried over anhydrous $Na_2SO_4$, filtered and concentrated *in vacuo.* Purification of the crude residue by flash chromatography on silica gel (elution with a gradient of 0% to 90% EtOAc/hexanes) followed by trituration with $Et_2O$/EtOAc afforded 45 mg (0.114 mmol; 61%) of title compound as an off-white solid. MS (ES) m/e 395 (M+1), 378 (M+H-$NH_3$), 453 (M+$AcO^-$).

| Elemental Analyses for $C_{23}H_{26}N_2O_4 \cdot 0.3H_2O$: | | | |
|---|---|---|---|
| Calculated | C, 69.08; | H, 6.71; | N, 7.01. |
| Found | C, 69.13; | H, 6.71; | N, 7.09. |

E. [9-[(Cyclohexyl)methyl]-5-carbamoylcarbazol-4-yl]oxyacetic acid

A slurry of [9-[(cyclohexyl)methyl]-5-carbamoylcarbazol-4-yl]oxyacetic acid, methyl ester (20 mg, 0.051 mmol) in 0.3 mL of THF and 0.1 mL of MeOH was treated with 0.1 mL of 1 N aq LiOH (0.1 mmol), and the mixture stirred at room temperature for 2 h. The reaction was acidified with 0.2 N HCl, and the organics were removed *in vacuo*. The white precipitate was filtered away from the aqueous layer and rinsed with $Et_2O$ to afford 16 mg (0.042 mmol; 83%) the title acid as a white powder. MS (ES) m/e 381 (M+1), 364 (M+H-$NH_3$), 379 (M-1).

| Elemental Analyses for $C_{22}H_{24}N_2O_4$: | | | |
|---|---|---|---|
| Calculated | C, 69.46; | H, 6.36; | N, 7.36. |
| Found | C, 69.34; | H, 6.35; | N, 7.29. |

EXAMPLE 14

Preparation of [9-[(Cyclopentyl)methyl]-5-carbamoylcarbazol-4-yl]oxyacetic acid

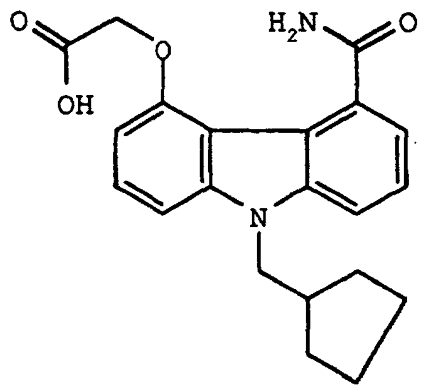

A. 9-[(Cyclopentyl)methyl]-5-carbomethoxy-1,2-dihydrocarbazol-4(3H)-one

A suspension of 5-carbomethoxy-1,2-dihydro-9H-carbazol-4(3H)-one (820 g, 3.37 mmol), a catalytic amount of NaI (ca. 10 mg) and $K_2CO_3$ (930 mg, 6.74 mmol) in 6 mL of DMF was treated with cyclopentylmethyl chloride *(JOC,* 1964, *29*, 421-423; 400 mg, 3.37 mmol). After stirring overnight at ambient temperature, an additional 800 mg of cyclopentylmethyl chloride and 1 g of NaI were added, and the resulting mixture was heated at 80 °C overnight. An additional 800 mg of cyclopentylmethyl chloride and 2.2 g of $Cs_2CO_3$ were added, and the reaction mixture was heated at 80 °C for 24 h. An additional 1.6 g of cyclopentylmethyl chloride was added, and the reaction mixture was heated at 80 °C for 3 d The mixture was poured into $H_2O$ (30 mL) and extracted with EtOAc (3 x 10 mL). The combined organic layers were dried over anhydrous $Na_2SO_4$, filtered, and concentrated *in vacuo.* The residue was purified by radial chromatography on silica gel (elution with gradient of 10% to 40% EtOAc/hexanes) to afford 775 mg (2.38 mmol; 71%) of 9-[(cyclopentyl)methyl]-5-carbomethoxy-1,2-dihydrocarbazol-4(3H)-one as a brown foam. MS (ES) m/e 326 (M+1), 384 ($M+AcO^-$).

| Elemental Analyses for $C_{20}H_{23}NO_3$: | | | |
|---|---|---|---|
| Calculated | C, 73.82; | H, 7.12; | N, 4.30. |
| Found | C, 74.12; | H, 7.21; | N, 4.45. |

B. 9-[(Cyclopentyl)methyl]-4-hydroxy-5-carbomethoxy carbazole

A solution of 9-[(cyclopentyl)methyl]-5-carbomethoxy-1,2-dihydrocarbazol-4(3H)-one (730 mg, 2.24 mmol) and 2,3-dichloro-5,6-dicyano-1,4-benzoquinone (560 mg, 2.47 mmol) in 20 mL of toluene was heated at 80 °C for 3 hours. The mixture was purified directly by column chromatography on silica gel (elution with $CH_2Cl_2$) to afford 140 mg (0.433 mmol; 19%) of 9-[(cyclopentyl)methyl]-4-hydroxy-5-carbomethoxy carbazole as a yellow oil which slowly solidified. MS (ES) m/e 324 (M+1), 322 (M-1).

| Elemental Analyses for $C_{20}H_{21}NO_3 \cdot 0.3H_2O$: | | | |
|---|---|---|---|
| Calculated | C, 73.06; | H, 6.62; | N, 4.26. |
| Found | C, 73.19; | H, 6.44; | N, 4.40. |

C. 9-[(Cyclopentyl)methyl]-4-hydroxy-5-carbamoyl carbazole

A solution of 9-[(cyclopentyl)methyl]-4-hydroxy-5-carbomethoxy carbazole (110 mg, 0.34 mmol) in 3 mL of THF and 20 mL ofconcentrated aqueous ammonium hydroxide was treated with a stream of NH3 gas to ensure saturation. The reaction vessel was capped, and the mixture heated to 35 °C with stirring until tlc indicated complete consumption of starting material (20 h). The THF was evaporated, and the aqueous layer was filtered. The resultant

solid was triturated with ether to afford 50 mg (0.162; 48%) of the title compound as a greenish-white solid. IR (KBr, $cm^{-1}$) 3416, 3199, 3126, 1630, 1599. 1442, 1262, 778. 'FAB HRMS m/e, Calcd for $C_{20}H_{21}N_2O_2$: 309.1603. Found: 309.1607 (M+1).'

D. [9-[(Cyclopentyl)methyl]-5-carbamoylcarbazol-4-yl]oxyacetic acid, methyl ester

A mixture of 9-[(cyclopentyl)methyl]-4-hydroxy-5-carbamoyl carbazole (45 mg, 0.146 mmol) and $CS_2CO_3$ (120 mg; 0.365 mmol) in 2 mL of DMF was treated with methyl bromoacetate (0.018 mL; 0.19 mmol). The reaction was stirred for 2 hours at ambient temperature, then it was diluted with EtOAc and $H_2O$ (10 mL each). The aqueous layer was saturated with solid Nacl extracted with EtOAc (2 x 10 mL). The combined organic layers were washed with $H_2O$ (2 x 25 mL), dried over anhydrous $Na_2SO_4$, filtered and concentrated *in vacuo*. Purification of the crude residue by flash chromatography on silica gel (elution with a gradient of 0% to 100% EtOAc/hexanes) followed by trituration with $Et_2O$/EtOAc afforded 26 mg (0.0683 mmol; 47%) of title compound as a tan solid. MS (ES) m/e 381 (M+1), 364 (M+H-$NH_3$), 439 (M+$AcO^-$).

| Elemental Analyses for $C_{23}H_{26}N_2O_4 \cdot 0.1H_2O$: | | | |
|---|---|---|---|
| Calculated | C, 69.13; | H, 6.38; | N, 7.33. |
| Found | C, 68.99; | H, 6.39; | N, 7.41. |

E. [9-[(Cyclopentyl)methyl]-5-carbamoylcarbazol-4-yl]oxyacetic acid

A slurry of [9-[(cyclopentyl)methyl]-5-carbamoylcarbazol-4-yl}oxyacetic acid, methyl ester (20 mg, 0.065 mmol) in 0.3 mL of THF and 0.1 mL of MeOH was treated with 0.1 mL of 1 N aq LiOH (0.1 mmol), and the mixture stirred at room temperature for 2 hours. The reaction was acidified with 0.2 N HCl, and the organics were removed *in vacuo.* The white precipitate was filtered away from the aqueous layer and rinsed with $Et_2O$ to afford 15 mg (0.0409 mmol; 63%) the title acid as a white powder. MS (ES) m/e 367 (M+1), 350 (M+H-$NH_3$), 365 (M-1).

| Elemental Analyses for $C_{21}H_{22}N_2O_4 \cdot 0.3H_2O$: | | | |
|---|---|---|---|
| Calculated | C, 67.84; | H, 6.13; | N, 7.53. |
| Found | C, 67.73; | H, 5.97; | N, 7.70. |

The compounds described herein are believed to achieve their beneficial therapeutic action principally by direct inhibition of human $sPLA_2$, and not by acting as antagonists for arachidonic acid, nor other active agents below arachidonic acid in the arachidonic acid cascade, such as 5-lipoxygenases, cyclooxygenases, etc. The method for inhibiting $sPLA_2$ mediated release of fatty acids comprises contacting $sPLA_2$ with an therapeutically effective amount of a compound of Formula (I) selected from the group consisting [9-benzyl-5-carbamoyl-1-fluorocarbazol-4-yl]oxyacetic acid, {9-[(phenyl)methyl]-5-carbamoylcarbazol-4-yl}oxyacetic acid, {9-[(3-fluorophenyl) methyl]-5-carbamoylcarbazol-4-yl}oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl}oxyacetic acid, sodium salt, {9-[(2-methylphenyl)methyl]-5-carbamoylcarbazol-4-yl}oxyacetic acid, sodium salt, {9-[(3-methylphenyl)methyl]-5-carbamoylcarbazol-4-yl}oxyacetic acid, sodium salt, {9-[(3-trifluoromethoxyphenyl)methyl]-5-carbamoylcarbazol-4-yl}oxyacetic acid, sodium salt, [9-benzyl-5-carbamoyl-1-chlorocarbazol-4-yl]oxyacetic acid, [9-[(cyclohexyl)methyl]-5-carbamoylcarbazol-4-yl]oxyacetic acid, [9-[(cyclopentyl)methyl]-5-carbamoylcarbazol-4-yl]oxyacetic acid or its pharmaceutically acceptable salt.

The compounds of the invention may be used in a method of treating a mammal (e.g., a human) to alleviate the pathological effects of septic shock, adult respiratory distress syndrome, pancreatitus, trauma, bronchial asthma, allergic rhinitis, and rheumatoid arthritis; wherein the method comprises administering to the mammal a compound of formula (I) in a therapeutically effective amount. A "therapeutically effective" amount is an amount sufficient to inhibit sPLA2 mediated release of fatty acid and to thereby inhibit or prevent the arachidonic acid cascade and its deleterious products. The therapeutic amount of compound of the invention needed to inhibit $sPLA_2$ may be readily determined by taking a sample of body fluid and assaying it for $sPLA_2$ content by conventional methods.

Throughout this document, the person or animal to be treated will be described as a "mammal", and it will be understood that the most preferred subject is a human. However it must be noted that the study of adverse conditions of the central nervous system in non-human animals is only now beginning, an that some instances of such treatments are coming into use. Accordingly, use of the present compounds in non-human animals is contemplated. It will be understood that the dosage ranges for other animals will necessarily be quite different from the doses administered to humans, and accordingly that the dosage ranges described be recalculated. For example, a small dog may be only $1/10^{th}$ of a typical human's size, and it will therefore be necessary for a much smaller dose to be

used. The determination of an effective amount for a certain non-human animal is carried out in the same manner described below in the case of humans, and veterinarians are well accustomed to such determinations.

As previously noted the compounds of this invention are useful for inhibiting $sPLA_2$ mediated release of fatty acids such as arachidonic acid. By the term, "inhibiting" is meant the prevention or therapeutically significant reduction in release of $sPLA_2$ initiated fatty acids by the compounds of the invention. By "pharmaceutically acceptable" it is meant the carrier, diluent or excipient must be compatible with the other ingredients of the formulation and not deleterious to the recipient thereof.

In general, the compounds of the invention are most desirably administered at a dose that will generally afford effective results without causing any serious side effects and can be administered either as a single unit dose, or if desired, the dosage may be divided into convenient subunits administered at suitable times throughout the day.

The specific dose of a compound administered according to this invention to obtain therapeutic or prophylactic effects will, of course, be determined by the particular circumstances surrounding the case, including, for example, the route of administration, the age, weight and response of the individual patient, the condition being treated and the severity of the patient's symptoms. Typical daily doses will contain a non-toxic dosage level of from about 0.01 mg/kg to about 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

A "chronic" condition means a deteriorating condition of slow progress and long continuance. As such, it is treated when it is diagnosed and continued throughout the course of the disease. An "acute" condition is an exacerbation of short course followed by a period of remission. In an acute event, compound is administered at the onset of symptoms and discontinued when the symptoms disappear.

Pancreatitis, trauma-induced shock, bronchial asthma, allergic rhinitis and rheumatoid arthritis may occur as an acute event or a chronic event. Thus, the treatment of these conditions contemplates both acute and chronic forms. Septic shock and adult respiratory distress, on the other hand, are acute conditions treated when diagnosed.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the compounds of the invention together with a pharmaceutically acceptable carrier or diluent therefor. The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from about 1 to about 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol. Other compositions can be made by dispersing the finely divided active ingredient in aqueous starch or sodium carboxymethyl cellulose solution or in a suitable oil.

The following pharmaceutical formulations 1 through 8 are illustrative only and are not intended to limit the scope of the invention in any way. "Active ingredient", refers to a compound according to Formula (I) or a pharmaceutically acceptable salt, solvate, or prodrug thereof.

Formulation 1

Hard gelatin capsules are prepared using the following ingredients:

| | Quantity (mg/capsule) |
|---|---|
| Compound of Example 1 | 250 |
| Starch, dried | 200 |
| Magnesium stearate | 10 |
| Total | 460 mg |

Formulation 2

A tablet is prepared using the ingredients below:

| | Quantity (mg/tablet) |
|---|---|
| Compound of Example 2 | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

Formulation 3

An aerosol solution is prepared containing the following components:

| | Weight |
|---|---|
| Compound of Example 3 | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

| | |
|---|---|
| Compound of Example 4 | 60 mg |
| Starch | 45 mg |

(continued)

| | |
|---|---|
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| Compound of Example 5 | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Compound of Example 6 | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| Compound of Example 7 | 50 mg |
| Sodium carboxymethyl cellulose | 50 mg |
| Syrup | 1.25 ml |
| Benzoic acid solution | 0.10 ml |
| Flavor | q.v. |

(continued)

| | |
|---|---|
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Compound of Example 8 | 100 mg |
| Isotonic saline | 1,000 ml |

The solution of the above ingredients generally is administered intravenously to a subject at a rate of 1 ml per minute.

Assay Experiments

Assay Example 1

The following chromogenic assay procedure was used to identify and evaluate inhibitors of recombinant human secreted phospholipase $A_2$. The assay described herein has been adapted for high volume screening using 96 well microtiter plates. A general description of this assay method is found in the article, "Analysis of Human Synovial Fluid Phospholipase $A_2$ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992 (the disclosure of which is incorporated herein by reference):

Reagents:

REACTION BUFFER -

$CaCl_2.2H_2O$ (1.47 g/L)
KCl (7.455 g/L)
Bovine Serum Albumin (fatty acid free) (1 g/L)
(Sigma A-7030, product of Sigma Chemical Co. St. Louis MO, USA)
TRIS HCl (3.94 g/L)
pH 7.5 (adjust with NaOH)

ENZYME BUFFER -

0.05 $NaOAc.3H_2O$, pH 4.5
0.2 NaCl
Adjust pH to 4.5 with acetic acid

DTNB -

5,5'-dithiobis-2-nitrobenzoic acid

RACEMIC DIHEPTANOYL THIO - PC

racemic 1,2-bis(heptanoylthio)-1,2-dideoxy-*sn*-glycero-3-phosphorylcholine
TRITON X-100TM prepare at 6.249 mg/ml in reaction buffer to equal 10uM
TRITON X-100TM is a polyoxy ethylene non-ionic detergent supplied by Pierce Chemical Company, 3747 N. Meridian Road, Rockford, Illinois 61101.

REACTION MIXTURE -

A measured volume of racemic dipheptanoyl thio PC supplied in chloroform at a concentration of 100 mg/ml is taken to dryness and redissolved in 10 millimolar TRITON X-100TM nonionic detergent aqueous solution. Reaction Buffer is added to the solution, then DTNB to give the Reaction Mixture.
The reaction mixture thus obtained contains 1mM diheptanoly thio-PC substrate, 0.29 mm Triton X-100TM detergent, and 0.12 mm DTMB in a buffered aqueous solution at pH 7.5.

Assay Procedure:

1. Add 0.2 ml reaction mixture to all wells;
2. Add 10 ul test compound (or solvent blank) to appropriate wells, mix 20 seconds;
3. Add 50 nanograms of $sPLA_2$ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, $IC_{50}$ values were determined. Typically, the $IC_{50}$ values were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of $IC_{50}$ values. $IC_{50}$ were determined by plotting log concentration versus inhibition values in the range from 10-90% inhibition.
Compounds of the instant invention were tested in Assay Example 1 and were found to be effective at concentrations of less than 100µM.

Assay Example 2

Method:

Male Hartley strain guinea pigs (500-700g) were killed by cervical dislocation and their heart and lungs removed intact and placed in aerated (95% $O_2$:5% $CO_2$) Krebs buffer. Dorsal pleural strips (4x1x25mm) were dissected from intact parenchymal segments (8x4x25mm) cut parallel to the outer edge of the lower lung lobes. Two adjacent pleural strips, obtained from a single lobe and representing a single tissue sample, were tied at either end and independently attached to a metal support rod. One rod was attached to a Grass force-displacement transducer Model FTO3C, product of Grass Medical Instruments Co., Quincy, MA, USA). Changes in isometric tension were displayed on a monitor and thermal recorder (product of Modular Instruments, Malvern, PA). All tissues were placed in 10 ml jacketed tissue baths maintained at 37°C. The tissue baths were continuously aerated and contained a modified Krebs solution of the following composition (millimolar) NaCl, 118.2; KCl, 4.6; $CaCl_2 \cdot 2H_2O$, 2.5; $MgSO_4 \cdot 7H_2O$, 1.2; $NaHCO_3$, 24.8; $KH_2PO_4$, 1.0; and dextrose, 10.0. Pleural strips from the opposite lobes of the lung were used for paired experiments. Preliminary data generated from tension/response curves demonstrated that resting tension of 800mg was optimal. The tissues were allowed to equilibrate for 45 min. as the bath fluid was changed periodically.

Cumulative concentration-response curves:

Initially tissues were challenged 3 times with KCl (40 mM) to test tissue viability and to obtain a consistent response. After recording the maximal response to KCl, the tissues were washed and allowed to return to baseline before the next challenge. Cumulative concentration-response curves were obtained from pleural strips by increasing the agonist concentration ($sPLA_2$) in the tissue bath by half-$log_{10}$ increments while the previous concentration remained in contact with the tissues (Ref.l, supra.). Agonist concentration was increased after reaching the plateau of the contraction elicited by the preceding concentration. One concentration-response curve was obtained from each tissue. To minimize variability between tissues obtained from different animals, contractile responses were expressed as a percentage of the maximal response obtained with the final KCl challenge. When studying the effects of various drugs on the contractile effects of $sPLA_2$, the compounds and their respective vehicles were added to the tissues 30 minutes prior to starting the $sPLA_2$ concentration-response curves.

Statistical analysis:

Data from different experiments were pooled and presented as a percentage of the maximal KCl responses (mean ± S.E.). To estimate the drug induced rightward shifts in the concentration response curves, the curves were analyzed simultaneously using statistical nonlinear modeling methods similar to those described by Waud (1976), Equation 26, p. 163, (Ref.2). The model includes four parameters: the maximum tissue response which was assumed the same for each curve, the $ED_{50}$ for the control curve, the steepness of the curves, and the $pA_2$, the concentration of antagonist that requires a two-fold increase in agonist to achieve an equivalent response. The Schild slope was determined to be 1, using statistical nonlinear modeling methods similar to those described by Waud (1976), Equation 27, p. 164 (Ref. 2). The Schild slope equal to 1 indicates the model is consistent with the assumptions of a competitive antagonist; therefore, the pA2 may be interpreted as the apparent $K_B$, the dissociation constant of the inhibitor.

To estimate the drug-induced suppression of the maximal responses, $sPLA_2$ responses (10 ug/ml) were determined in the absence and presence of drug, and percent suppression was calculated for each pair of tissues. Representative examples of inhibitory activities are presented in Table 2, below.

Ref. 1 - Van, J.M.: Cumulative dose-response curves. II. Technique for the making of dose-response curves in isolated organs and the evaluation of drug parameters. Arch. Int. Pharmacodyn. Ther., 143: 299-330, 1963.

Ref. 2 - Waud, D.: Analysis of dose-response relationships. in Advances in General and Cellular Pharmacology eds Narahashi, Bianchi 1:145-178, 1976.

Compounds of the instant invention were tested in Assay Example 2 and were found to be effective at concentrations below 20μM.

Assay Example 3 $sPLA_2$ Transgenic Mice Assay

Materials & Methods

The mice utilized in these studies were mature, 6-8 month old, $ZnSO_4$-stimulated, hemizygous line 2608[a] transgenic mice (Fox et. al. 1996). Transgenic mice from this line express human $sPLA_2$ in the liver and other tissues and typically achieve levels of human $sPLA_2$ in their circulation of approximately 173 ± 10 ng/ml when maximally stimulated with $ZnSO_4$ (Fox, *et al.* 1996). The mice were housed under constant humidity and temperature and received food and water ad libitum. Animal room lighting was maintained on a 12-hour light/dark cycle and all experiments were performed at the same time of the day during the early morning light period.

For intravenous testing, compounds or vehicle were administered as an IV bolus via the tail vein in a volume of 0.15 ml. Vehicle consisted of 1-5% dimethylsulfoxide, 1-5% ethanol and 10-30% polyethylene glycol 300 in $H_2O$; the concentrations of these ingredients were adjusted according to the solubility of the compound. Mice were bled retro-orbitally prior to drug or vehicle administration and 30 minutes, 2 and 4 hours thereafter. Three to six mice were used for each dose. $PLA_2$ catalytic activity in the serum was assayed with a modified phosphatidylcholine/deoxycholine mixed micelle assay (Fox, *et al*. 1996, Schadlich, *et al.,* 1987) utilizing 3 mM sodium deoxycholate and 1 mM 1-palmitoyl-2-oleoyl-sn-glycero-3-phosphocholine.

For oral testing, compounds were dissolved in 1-5% ethanol/10-30% polyethylene glycol 300 in $H_2O$ or were suspended in 5% dextrose in $H_2O$ and administered by oral gavage. Serum was prepared from retro-orbital blood and assayed for $PLA_2$ catalytic activity as above.

References

Fox, N., M. Song, J. Schrementi, J. D. Sharp, D. L. White, D. W. Snyder, L. W. Hartley, D. G. Carlson, N. J. Bach, R. D. Dillard, S. E. Draheim, J. L. Bobbitt, L. Fisher and E. D. Mihelich. 1996. Eur. J. Pharmacol. 308: 195.

Schadlich, H.R., M. Buchler, and H. G. Beger, 1987, J. Clin. Chem. Clin. Biochem. 25, 505.

Compounds of the instant invention were tested in Assay Example 3 and were found to be effective.

While the present invention has been illustrated above by certain specific embodiments, it is not intended that these specific examples should limit the scope of the invention as described in the appended claims.

## Claims

1. A compound selected from the group consisting of [9-benzyl-5-carbamoyl-1-fluorocarbazol-4-yl]oxyacetic acid, {9-[(phenyl)methyl]-5-carbamoylcarbazol-4-yl}oxyacetic acid, {9-[(3-fluorophenyl)methyl]-5-carbamoylcarbazol-4-yl}oxyacetic acid, {9-[(3-chlorophenyl)methyl]-5-carbamoylcarbazol-4-yl}oxyacetic acid, {9-[(3-trifluoromethylphenyl)methyl]-5-carbamoylcarbazol-4-yl}oxyacetic acid, sodium salt, {9-[(2-methylphenyl)methyl]-5-carbamoylcarbazol-4-yl}oxyacetic acid, sodium salt, {9-[(3-methylphenyl)methyl]-5-carbamoylcarbazol-4-yl}oxyacetic acid sodium salt, {9-[(3-trifluoromethoxyphenyl)methyl]-5-carbamoylcarbazol-4-yl}oxyacetic acid sodium salt, [9-benzyl-5-carbamoyl-1-chlorocarbazol-4-yl]oxyacetic acid, [9-[(cyclohexyl)methyl]-5-carbamoylcarbazol-4-yl]oxyacetic acid, and [9-[(cyclopentyl)methyl]-5-carbamoylcarbazol-4-yl]oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

2. A compound of **Claim 1** which is {9-[(phenyl)methyl]-5-carbamoylcarbazol-4-yl}oxyacetic acid, or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

3. A compound of **Claim 1** which is of [9-benzyl-5-carbamoyl-1-fluorocarbazol-4-yl]oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

4. A compound of **Claim 1** which is {9-[(3-fluorophenyl)methyl]-5-carbamoylcarbazol-4-yl}oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

5. A compound of **Claim 1** which is {9-[(3-chlorophenyl)methyl]-5-carbamoylcarbazol-4-yl}oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

6. A compound of **Claim 1** which is {9-[(3-trifluoromethylphenyl)methyl]-5-carbamoylcarbazol-4-yl}oxyacetic acid, sodium salt or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt thereof.

7. A compound of **Claim 1** which is {9-[(2-methylphenyl)methyl]-5-carbamoylcarbazol-4-yl}oxyacetic acid sodium salt or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt thereof.

**8.** A compound of **Claim 1** which is {9-[(3-methylphenyl)methyl]-5-carbamoylcarbazol-4-yl}oxyacetic acid sodium salt or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt thereof.

**9.** A compound of **Claim 1** which is {9-[(3-trifluoromethoxyphenyl)methyl]-5-carbamoylcarbazol-4-yl}oxyacetic acid sodium salt or a pharmaceutically acceptable racemate, solvate, tautomer, optical isomer, prodrug derivative selected from a methyl, ethyl, propyl, isopropyl, butyl, sec-butyl, tert-butyl,morpholinoethyloxy, diethylglycolamide or diethylaminocarbonylmethoxy ester, or salt thereof.

**10.** A compound of **Claim 1** which is [9-benzyl-5-carbamoyl-1-chlorocarbazol-4-yl]oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

**11.** A compound of **Claim 1** which is [9-[(cyclohexyl)methyl]-5-carbamoylcarbazol-4-yl]oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

**12.** A compound of **Claim 1** which is [9-[(cyclopentyl)methyl]-5-carbamoylcarbazol-4-yl]oxyacetic acid or a pharmaceutically acceptable racemate, solvate, tautomer, optical isomer, prodrug derivative selected from a methyl, ethyl, propyl, isopropyl, butyl, sec-butyl, tert-butyl, morpholinoethyloxy, diethylglycolamide or diethylaminocarbonylmethoxy ester, or salt, thereof.

**13.** A compound as claimed in any one of **Claims 1 to 12** wherein the prodrug derivative is a methyl, ethyl, propyl, isopropyl, butyl, morpholinoethyloxy or diethylglycolamide ester.

**14.** A compound as claimed in any one of **Claims 1 to 5 and 10 to 12** wherein the salt is sodium.

**15.** A pharmaceutical formulation comprising a compound as claimed in any one of **Claims 1 to 12** together with a pharmaceutically acceptable carrier or diluent therefor.

**16.** A pharmaceutical formulation adapted for the treatment of a condition associated with inhibiting $sPLA_2$, containing a compound as claimed in any one of **Claims 1 to 12** together with a pharmaceutically acceptable carrier or diluent therefor.

**17.** The use of a compound of any one of **claims 1 to 12** for the manufacture of a medicament for selectively inhibiting $sPLA_2$ in a mammal.

**18.** The use of **Claim 17** wherein the mammal is a human.

**19.** The use of a compound as claimed in any one of **Claims 1 to 12** in the manufacture of a medicament adapted for the administration of said compound in an amount sufficient to inhibit $sPLA_2$ mediated release of fatty acid and to thereby inhibit or prevent the arachidonic acid cascade and its deleterious products, for alleviating the pathological effects of $sPLA_2$ related diseases.

**20.** The use of a compound as claimed in any one of **Claims 1 to 12** for the manufacture of a medicament for alleviating the pathological effects of $sPLA_2$ related diseases.

**21.** The use of a compound as claimed in any one of **Claims 1 to 12** for the manufacture of a medicament adapted for contacting $sPLA_2$ with said compound, for inhibiting $sPLA_2$.

**22.** The use of a compound as claimed in any one of **Claims 1 to 12** for the manufacture of a medicament for treating sepsis, septic shock, rheumatoid arthritis, osteoarthritis, stroke, apoptosis, asthma, chronic bronchitis, acute bronchitis, cystic fibrosis, inflammatory bowel disease, or pancreatitis.

**23.** The use of **claim 21** for alleviating the pathological effects of sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, Tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis; and related diseases.

**Patentansprüche**

**1.** Verbindung, ausgewählt aus der Gruppe, die besteht aus
[9-Benzyl-5-carbamoyl-1-fluorcarbazol-4-yl]oxyessigsäure,
{9-[(Phenyl)methyl]-5-carbamoylcarbazol-4-yl}oxyessigsäure,
{9-[(3-Fluorphenyl)methyl]-5-carbamoylcarbazol-4-yl}oxyessigsäure,
{9-[(3-Chlorphenyl)methyl]-5-carbamoylcarbazol-4-yl}oxyessigsäure,
{9-[(3-Trifluormethylphenyl)methyl]-5-carbamoylcarbazol-4-yl}oxyessigsäure-Natriumsalz,
{9-[(2-Methylphenyl)methyl]-5-carbamoylcarbazol-4-yl}oxyessigsäure-Natriumsalz,
{9-[(3-Methylphenyl)methyl]-5-carbamoylcarbazol-4-yl}oxyessigsäure-Natriumsalz,
{9-[(3-Trifluormethoxyphenyl)methyl]-5-carbamoylcarbazol-4-yl}oxyessigsäure-Natriumsalz,
[9-Benzyl-5-carbamoyl-1-chlorcarbazol-4-yl]oxyessigsäure,
[9-(Cyclohexyl)methyl]-5-carbamoylcarbazol-4-yl]oxyessigsäure und
[9-[(Cyclopentyl)methyl]-5-carbamoylcarbazol-4-yl]oxyessigsäure oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

**2.** Verbindung nach Anspruch 1, die {9-[(Phenyl)methyl]-5-carbamoylcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

**3.** Verbindung nach Anspruch 1, die [9-Benzyl-5-carbamoyl-1-fluorcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

**4.** Verbindung nach Anspruch 1, die {9-[(3-Fluorphenyl)methyl]-5-carbamoylcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

**5.** Verbindung nach Anspruch 1, die {9-[(3-Chlorphenyl)methyl]-5-carbamoylcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

**6.** Verbindung nach Anspruch 1, die {9-[(3-Trifluormethylphenyl)methyl]-5-carbamoylcarbazol-4-yl}oxyessigsäure-Natriumsalz ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

7. Verbindung nach Anspruch 1, die {9-[(2-Methylphenyl)methyl]-5-carbamoylcarbazol-4-yl}oxyessigsäure-Natriumsalz ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

8. Verbindung nach Anspruch 1, die {9-[(3-Methylphenyl)methyl]-5-carbamoylcarbazol-4-yl}oxyessigsäure-Natriumsalz ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

9. Verbindung nach Anspruch 1, die {9-[(3-Trifluormethoxyphenyl)methyl]-5-carbamoylcarbazol-4-yl}oxyessigsäure-Natriumsalz ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

10. Verbindung nach Anspruch 1, die [9-Benzyl-5-carbamoyl-1-chlorcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

11. Verbindung nach Anspruch 1, die [9-[(Cyclohexyl)methyl]-5-carbamoylcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

12. Verbindung nach Anspruch 1, die [9-[(Cyclopentyl)methyl]-5-carbamoylcarbazol-4-yl}oxyessigsäure ist oder ein pharmazeutisch annehmbares Razemat, Solvat, Tautomer, optisches Isomer, Prodrugderivat, ausgewählt aus einem Methyl-, Ethyl-, Propyl-, Isopropyl-, Butyl-, sek-Butyl-, tert-Butyl-, Morpholinoethyloxy-, Diethylglycolamid- oder Diethylaminocarbonylmethoxyester oder ein Salz hiervon.

13. Verbindung nach einem der Ansprüche 1 bis 12, worin das Produgderivat ein Methyl-, Ethyl-, Propyl-, Isopropyl-, Butyl-, Morpholinoethyloxy- oder Diethylglycolamidester ist.

14. Verbindung nach einem der Ansprüche 1 bis 5 und 10 bis 12, worin das Salz Natrium ist.

15. Pharmazeutische Formulierung, die eine Verbindung nach einem der Ansprüche 1 bis 12 zusammen mit einem pharmazeutisch annehmbaren Träger oder Verdünnungsmittel hierfür enthält.

16. Pharmazeutische Formulierung, die zur Behandlung eines Zustands angepasst ist, der mit der Hemmung der $sPLA_2$ assoziiert ist, die eine Verbindung nach einem der Ansprüche 1 bis 12 zusammen mit einem pharmazeutisch annehmbaren Träger oder Verdünnungsmittel hierfür enthält.

17. Verwendung einer Verbindung nach einem der Ansprüche 1 bis 12 zur Herstellung eines Arzneimittels zur selektiven Hemmung der $sPLA_2$ bei einem Säuger.

18. Verwendung nach Anspruch 17, worin der Säuger ein Mensch ist.

19. Verwendung einer Verbindung nach einem der Ansprüche 1 bis 12 zur Herstellung eines Arzneimittels, das zur Verabreichung dieser Verbindung in einer Menge angepasst ist, die zur Hemmung der $sPLA_2$ vermittelten Freisetzung von Fettsäuren und dadurch zur Hemmung oder Prävention der Arachidonsäurekaskade und ihrer schädlichen Produkte zur Linderung der pathologischen Effekte der mit $sPLA_2$ zusammenhängenden Erkrankungen ausreichend ist.

20. Verwendung einer Verbindung nach einem der Ansprüche 1 bis 12 zur Herstellung eines Arzneimittels zur Linderung der pathologischen Effekte der mit $sPLA_2$ zusammenhängenden Erkrankungen.

21. Verwendung einer Verbindung nach einem der Ansprüche 1 bis 12 zur Herstellung eines Arzneimittels, das zur Kontaktierung von $sPLA_2$ mit dieser Verbindung zur Hemmung der $sPLA_2$ angepasst ist.

22. Verwendung einer Verbindung nach einem der Ansprüche 1 bis 12 zur Herstellung eines Arzneimittels zur Behandlung von Sepsis, septischem Schock, rheumatoider Arthritis, Osteoarthritis, Schlaganfall, Apoptose, Asthma, chronischer Bronchitis, akuter Bronchitis, cystischer Fibrose, entzündlicher Darmerkrankung oder Pankreatitis.

23. Verwendung nach Anspruch 21 zur Linderung der pathologischen Effekte von Sepsis, septischem Schock, Atemstresssyndrom beim Erwachsenen, Pankreatitis, Trauma induziertem Schock, Bronchialasthma, allergischer Rhinitis, rheumatoider Arthritis, cystischer Fibrose, Schlaganfall, akuter Bronchitis, chronischer Bronchitis, akuter Broncheolitis, chronischer Broncheolitis, Osteoarthritis, Gicht, Spondylarthropathie, alkylosierender Spondylitis, Reiter Syndrom, psoriatischer Arthropathie, enterapathischer Spondylitis, juveniler Arthropathie oderjuveniler alkylosierender Spondylitis, reaktiver Arthropathie, infektiöser oder postinfektiöser Arthritis, Gonokokkenarthritis, Tuberkulosearthritis, viraler Arthritis, pilzbedingter Arthritis, Syphilis-bedingter Arthritis, Lyme-Erkankung, Arthritis, die mit "vaskulitischen Syndromen" assoziiert ist, Polyarteritis nodosa, hyperempfindlicher Vaskulitis, Luegenec Granulomatose, Polymyalgia rheumatica, Gelenkszellarteritis, Arthropathie durch Calciumkristallablagerung, Pseudogicht, nichtartikulärem Rheuma, Bursitis, Tenosynovitis, Epicondylitis (Tennisellenbogen), Carpaltunnelsyndrom, Verletzung durch wiederkehrende Tätigkeit (Tippen), verschiedenen Formen der Arthritis, neuropatischer Gelenkserkrankung (Charcotgelenk und Gelenke), Hämarthrose (hämarthrotisch), Purpura Schönlein-Hennoch, hypertropher Osteoarthropathie, multizentrischer Reticulohistiocytose, Arthritis, die mit bestimmten Erkrankungen assoziiert ist, Surcoilose, Hämochromatose, Sichelzellerkrankung und anderen Hämoglobinopathien, Hyperlipoproteinämie, Hypogammaglobulinämie, Hyperparathyreoidismus, Akromegalie, familiärem Mittelmeerfieber, Behat Erkrankung, systemischem Lupus erythrematodes oder Polychondritisrückfall und verwandten Erkrankungen.

## Revendications

1. Composé choisi dans le groupe constitué de l'acide [9-benzyl-5-carbamoyl-1-fluorocarbazol-4-yl]oxyacétique, de l'acide {9-[(phényl)méthyl]-5-carbamoylcarbazol-4-yl}oxyacétique, de l'acide {9-[(3-fluorophényl)méthyl]-5-carbamoylcarbazol-4-yl}oxyacétique, de l'acide {9-[(3-chlorophényl)méthyl]-5-carbamoylcarbazol-4-yl}oxyacétique, du sel sodique de l'acide {9-[(3-trifluoro-méthylphényl)méthyl]-5-carbamoylcarbazol-4-yl}oxyacétique, du sel sodique de l'acide {9-[(2-méthylphényl)rnéthyl]-5-carbamoylcarbazol-4-yl}oxyacétique, du sel sodique de l'acide {9-[(3-méthylphényl)méthyl]-5-carbamoylcarbazol-4-yl}oxyacétique, du sel sodique de l'acide {9-[(3-trifluorométhoxyphényl) méthyl]-5-carbamoylcarbazol-4-yl}oxyacétique, de l'acide [9-benzyl-5-carbamoyl-1-chlorocarbazol-4-yl]oxyacétique, de l'acide [9-[(cyclohexyl)méthyl]-5-carbamoylcarbazol-4-yl]oxyacétique et de l'acide [9-[(cyclopentyl)méthyl]-5-carbamoylcarbazol-4-yl]oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

2. Composé selon la revendication 1 qui est l'acide {9-[(phényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

3. Composé selon la revendication 1 qui est l'acide [9-benzyl-5-carbamoyl-1-fluorocarbazol-4-yl]oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

4. Composé selon la revendication 1 qui est l'acide {9-[(3-fluorophényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

5. Composé selon la revendication 1 qui est l'acide {9-[(3-chlorophényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

6. Composé selon la revendication 1 qui est le sel sodique de l'acide {9-[(3-trifluorométhylphényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

7. Composé selon la revendication 1 qui est le sel sodique de l'acide {9-[(2-méthylphényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

8. Composé selon la revendication 1 qui est le sel sodique de l'acide {9-[(3-méthylphényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

9. Composé selon la revendication 1 qui est le sel sodique de l'acide {9-[(3-trifluorométhoxyphényl)méthyl]-5-carbamoylcarbazol-4-yl}oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

10. Composé selon la revendication 1 qui est l'acide [9-benzyl-5-carbamoyl-1-chlorocarbazol-4-yl]oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

11. Composé selon la revendication 1 qui est l'acide [9-[(cyclohexyl)méthyl]-5-carbamoylcarbazol-4-yl]oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

12. Composé selon la revendication 1 qui est l'acide [9-[(cyclopentyl)méthyl]-5-carbamoylcarbazol-4-yl]oxyacétique ou un racémate, un produit de solvatation, un tautomère, un isomère optique, un dérivé de pro-médicament choisi parmi un ester méthylique, éthylique, propylique, isopropylique, butylique, sec-butylique, tert-butylique, morpholinoéthyloxy, diéthylglycolamide ou diéthylaminocarbonylméthoxy ou un sel pharmaceutiquement acceptable de celui-ci.

13. Composé selon l'une quelconque des revendications 1 à 12, dans lequel le pro-médicament dérivé est un ester méthylique, éthylique, propylique, isopropylique, butylique, morpholinoéthyloxy ou diéthylglycolamide.

14. Composé selon l'une quelconque des revendications 1 à 5 et 10 à 12, dans lequel le sel est le sodium.

15. Formulation pharmaceutique comprenant un composé selon l'une quelconque des revendications 1 à 12 et un support ou un diluant pharmaceutiquement acceptable.

16. Formulation pharmaceutique adaptée au traitement d'une pathologie liée à l'inhibition de $sPLA_2$, comprenant un composé selon l'une quelconque des revendications 1 à 12 et un support ou un diluant pharmaceutiquement acceptable.

17. Utilisation d'un composé selon l'une quelconque des revendications 1 à 12 pour la fabrication d'un médicament destiné à inhiber sélectivement $sPLA_2$ chez un mammifère.

18. Utilisation selon la revendication 17, dans laquelle le mammifère est un être humain.

**19.** Utilisation d'un composé selon l'une quelconque des revendications 1 à 12 pour la fabrication d'un médicament adapté pour l'administration dudit composé en une quantité suffisante pour inhiber la libération médiée par $sPLA_2$ d'acide gras et pour ainsi inhiber ou empêcher la cascade de l'acide arachidonique et ses produits nuisibles, afin de soulager les effets pathologiques de maladies liées à $sPLA_2$.

**20.** Utilisation d'un composé selon l'une quelconque des revendications 1 à 12 pour la fabrication d'un médicament destiné à soulager les effets pathologiques de maladies liées à $sPLA_2$.

**21.** Utilisation d'un composé selon l'une quelconque des revendications 1 à 12 pour la fabrication d'un médicament adapté pour mettre en contact $sPLA_2$ avec ledit composé, afin d'inhiber $sPLA_2$.

**22.** Utilisation d'un composé selon l'une quelconque des revendications 1 à 12 pour la fabrication d'un médicament destiné à traiter une septicémie, un choc septique, une polyarthrite rhumatoïde, une arthrose, une attaque, une apoptose, un asthme, une bronchite chronique, une bronchite aiguë, une fibrose kystique, une maladie du côlon inflammatoire ou une pancréatite.

**23.** Utilisation selon la revendication 21 pour soulager les effets pathologiques d'une septicémie, d'un choc septique, d'un syndrome de détresse respiratoire aigu de l'adulte, d'une pancréatite, d'un choc provoqué par un traumatisme, d'un asthme bronchique, d'une rhinite allergique, d'une polyarthrite rhumatoïde, d'une fibrose kystique, d'une attaque, d'une bronchite aiguë, d'une bronchite chronique, d'une bronchiolite aiguë, d'une bronchiolite chronique, d'une arthrose, d'une goutte, d'une spondylarthropathie, d'une pelvispondylite rhumatismale, du syndrome de Reiter, d'une arthropathie psoriasique, d'une spondylite entéropathique, d'une arthropathie juvénile, ou d'une pelvispondylite rhumatismale juvénile, d'une arthropathie réactive, d'une arthrite infectieuse ou post-infectieuse, d'une arthrite gonococcique, d'une arthrite tuberculeuse, d'une arthrite virale, d'une arthrite fongique, d'une arthrite syphilique, de la maladie de Lyme, d'une arthrite associée aux « syndromes vasculitiques », d'une périartérite noueuse, d'une angéite d'hypersensibilité, d'une granulomatose de Luegenec, d'une pseudopolyarthrite rhizomélique, d'une artérite cellulaire des articulations, d'une arthropathie liée à un dépôt de cristaux de calcium, d'une chondrocalcinose articulaire, d'une pseudo-goutte, d'un rhumatisme non articulaire, d'une bursite, d'une ténosynovite, d'une épicondylite (« tennis elbow : épicondylite des joueurs de tennis), du syndrome du canal carpien, d'un trouble lié à une utilisation répétée (dactylographie), de diverses formes d'arthrite, d'une maladie des articulations névrotiques (charco et articulations), d'une hémarthrose (hémarthrosique), d'un purpura rhumatoïde de Henoch-Schonlein, d'une ostéo-arthropathie hypertrophiante, d'une réticulo-histiocytose multicentrique, d'une arthrite associée à certaines maladies, d'une surcoilose, d'une hémochromatose, d'une drépanocyte et d'autres hémoglobinopathies, d'une hyperlipidémie, d'une hypogammaglobulinémie, d'une hyperparathyroïdie, d'une acromégalie, d'une brucellose familiale, de la maladie de Behat, d'un lupus érythémateux disséminé ou d'une polychondrite atrophiante ; et d'autres maladies apparentées.